# EUROPEAN PATENT APPLICATION

(11) **EP 4 736 878 A2**
(43) Date of publication of application: **06.05.2026**
(21) Application number: 26152316.1
(22) Date of filing: 08.06.2021
(51) Int. Cl.: A61K 45/06

(54) **SATRAPLATIN FOR USE IN THE TREATMENT OF LYMPHOID NEOPLASMS**

(30) Priority: 08.07.2020 EP 20184760
(62) Divisional of application: 21730609.1
(71) Applicant: pharma& Schweiz GmbH, 6330 Cham (CH)
(72) Inventor: RENNER, Christoph Robert, 6312 Steinhausen, Zug (CH); ZANDER, Thilo Joachim, 6312 Steinhausen, Zug (CH); MARKSON, Gabriel Benjamin, 6312 Steinhausen, Zug (CH); DAHM, Felix, 6312 Steinhausen, Zug (CH)
(74) Representative: HGF

(57) **Abstract**

The present invention relates to satraplatin for use in a method of treating a malignancy of the lymphoid tissue in a mammal, preferably a human, and in particular of a malignancy of the lymphoid tissue with primary or secondary central nervous system (CNS) manifestation such as primary diffuse large B cell lymphoma of the CNS.

## Description

The present invention relates to satraplatin for use in a method of treating a malignancy of the lymphoid tissue in a mammal, preferably in a human, and in particular of a malignancy of the lymphoid tissue with primary or secondary central nervous system (CNS) manifestation such as primary diffuse large B cell lymphoma of the CNS.

### RELATED ART

Malignancies of the lymphoid tissue are lymphoid neoplasms that derive from the lymphoid cell lines. The lymphoid cell line produces B, T, NK and plasma cells. Well characterised lymphoid neoplasms include lymphoma and multiple myeloma. Lymphomas are tumours that develop from lymphocytes of the lymph glands (lymph nodes) located throughout the body. Albeit forming and maturing in the lymphatic organs, lymphocytes have the physiological task of circulating in the bloodstream and in the various organs of the body in order to detect foreign antigens to be killed. Tumoral lymphocytes retain such capacity to circulate in the body, so that generally lymphomas are widespread throughout the body since their onset, also at a distance from the place of origin. Multiple myeloma affects plasma cells that accumulate in the bone marrow and often also affects several areas of the body, such as the spine, skull, pelvis and ribs. Thus, malignancies of the lymphoid tissue can spread, through the blood and/or lymphatic vessels, from the lymph nodes to other lymph nodes or organs, both lymphatic (bone marrow, spleen, etc.) and extra lymphatic (skin, lungs, central nervous system, stomach, kidney, liver and the like) (Vardiman JW et al. Blood (2009) 114:1787-1797). The WHO classification of hematopoietic and lymphoid tumors and the associated monograph, first published in 2001 and most recently updated in 2016, represent the established guidelines for the diagnosis of malignant lymphomas and multiple myeloma. The WHO classification places an emphasis on cell lineage and classifies the lymphoid neoplasms into the following groups: (i) mature B-cell lymphoid neoplasms, (ii) mature T and NK-cell neoplasms, (iii) Hodgkin lymphomas, (iv) post-transplant lymphoproliferative disorders (PTLD) and (v) histiocytic and dendritic cell neoplasms (Swerdlow SH et al. Blood (2016) 127:2375-2390). Lymphoma and multiple myeloma are mostly incurable diseases requiring long-term, sometimes permanent treatment.

The central nervous system (CNS) is an immunologically privileged site which is separated from the circulation by the blood-brain barrier and thus, lymphomas with CNS manifestation including primary or secondary CNS lymphomas are some of the most difficult to treat rare cancer entities. Primary central nervous system lymphoma (PCNSL), which is a highly aggressive non-Hodgkin lymphoma confined to the CNS including the brain, spine, eyes, and leptomeninges without evidence of systemic spread (Grommes C and DeAngelis LM J Clin Oncol (2017) 35:2410-2418), can only be cured in a subset of young/fit patients with extensive (immuno-) chemotherapy (Mendez JS et al. Neuro Oncol (2018) 20:687-694; Houillier C et al. Neurology (2020) 94:1027-1039). More than 70% of all patients relapse within 2 years and will die within 6-9 months after relapse. In addition, the frequency of CNS lymphoma is increasing worldwide with a particular increase in elderly patients (Shiels MS et al. Br J Haematol (2016) 174:417-424). Therefore, the treatment of malignancies of the lymphoid tissues with CNS manifestation, and in particular, the treatment of primary CNS lymphoma is of high unmet medical need.

Platinum-based compounds are a mainstay of cancer chemotherapy. Since their original discovery, platinum compounds have emerged as important agents for the therapy of several human malignancies including lung, head and neck, and cervical cancer. There are three platinum compounds that are predominantly used in the current treatment of cancer - cisplatin, carboplatin, and oxaliplatin. As presented in the Physicians' Cancer Chemotherapy Drug Manual (Edward Chu; Vincent T. DeVita Jr.Goyena R, Fallis A. Physicians' Cancer Chemotherapy Drug Manual, 2019) they all belong to the same class of platinum salts, but still they differ considerably in their efficacy, therapeutic use, pharmacokinetics, adverse effect profile and administration route.

Cisplatin is a cornerstone drug in cancer treatment. However, its use is usually limited due to severe, dose-limiting adverse effects such as neurotoxicity, ototoxicity and renal toxicity. Moreover, cisplatin has to be administered intravenously (Bhargava A and Vaishampayan UN Expert Opin Investig Drugs (2009) 18:1787-1797; Akshintala S et al. Pediatr Blood Cancer (2015) 62:603-610).These disadvantages limit the use of cisplatin to certain patient populations and disease indications.

There is a real need for novel treatment options for cancer patients including those with lymphoid neoplasms such as lymphoma or multiple myeloma.

### SUMMARY OF THE INVENTION

Satraplatin has been developed and emerged as an oral platinum analogue with a better toxicity profile than cisplatin. Despite extensive efforts and numerous clinical trials, however, FDA approval has not yet been achieved (Choy H, Park C, Yao M Clin Cancer Res (2008) 14:1633-1638; Bhargava A and Vaishampayan UN Expert Opin Investig Drugs (2009) 18:1787-1797).

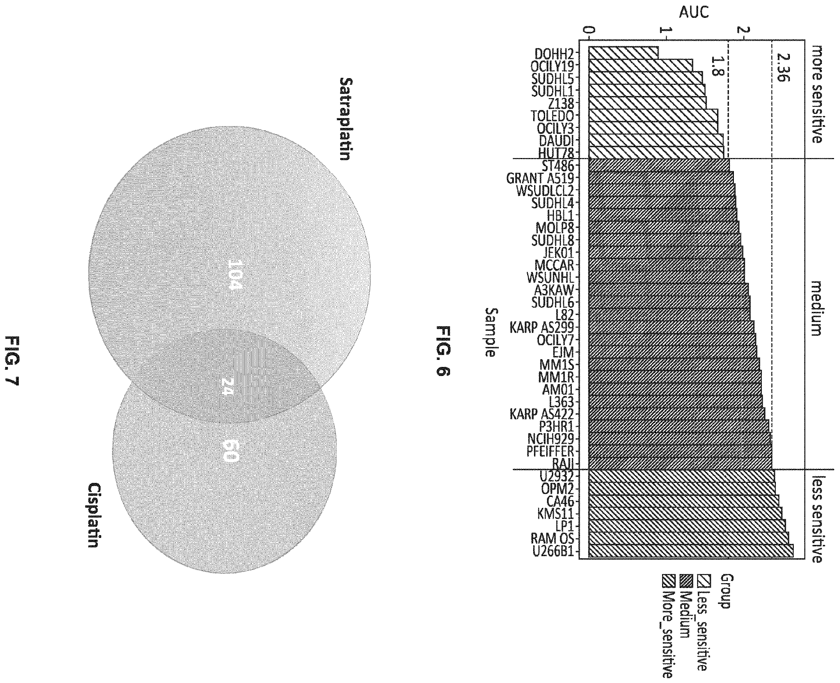

The inventors have completed a pathway analysis that confirms the difference in pathway activation and usage between satraplatin and cisplatin (see examples section below; see also Figure 7). These data clearly reveal that different pathways with differentially expressed genes contribute to the cell sensitivity (and thus treatment efficacy) associated with each of these platinum compounds.

Despite this, the inventors have surprisingly shown that satraplatin can effectively be used as an alternative to cisplatin to induce cell death of cell lines of lymphoid origin. The data presented herein shows that satraplatin is not only at least equivalent to cisplatin in killing lymphoma and multiple myeloma cell lines *in-vitro* - while none of the tested cell lines showed a resistance to satraplatin - but, furthermore, that satraplatin was effective in killing genetically heterogeneic lymphoma cell lines including those resembling primary or secondary CNS lymphoma. The inventors have therefore identified a novel means for treating lymphoid neoplasms generally, which is also effective on previously difficult to treat lymphoma with primary or secondary CNS manifestation.

Therefore, in a first aspect, the present invention provides satraplatin for use in a method of treating a malignancy of the lymphoid tissue in a mammal, preferably a human, while in a further aspect, the present invention provides satraplatin for use in a method of treating a malignancy of the lymphoid tissue in a mammal, preferably a human, wherein said malignancy is a malignancy of the lymphoid tissue with primary or secondary central nervous system (CNS) manifestation, wherein preferably said malignancy is a primary DLBCL of the central nervous system (CNS). The invention and in particular said aspects thereof are further supported by satraplatin's properties of being orally available and lipophilic with a clinically proven meaningful CNS penetration and sufficient blood-brain barrier penetration.

In another aspect, the present invention provides satraplatin for use in a method of treating a malignancy of the lymphoid tissue in a mammal, preferably a human, wherein the method comprising administering a therapeutically effective amount of satraplatin to said mammal, preferably said human.

In another aspect, the present invention provides a method of treating a malignancy of the lymphoid tissue in a mammal, preferably a human, wherein the method comprising administering a therapeutically effective amount of satraplatin to said mammal, preferably said human.

In a further aspect, the present invention provides the use of satraplatin in the manufacture of a medicament for the treatment of a malignancy of the lymphoid tissue in a mammal, preferably a human.

In again another aspect, the present invention provides an article of manufacture for treating a malignancy of the lymphoid tissue in a mammal, preferably a human comprising satraplatin and instructions for use.

In one aspect, the present invention provides satraplatin for use in treating a lymphoid neoplasm in a mammal.

Suitably, said mammal may be a human.

Suitably, said lymphoid neoplasm may be a mature B-cell neoplasm or a mature T-cell neoplasm.

Suitably, said mature B-cell neoplasm or mature T-cell neoplasm may be a lymphoma, optionally wherein the lymphoma is a lymphoma with CNS manifestation e.g. primary or secondary CNS lymphoma.

Suitably, said mature B-cell neoplasm or said mature T-cell neoplasm may be selected from the group consisting of: plasma cell myeloma (multiple myeloma), mantle cell lymphoma, Burkitt lymphoma, diffuse large B cell lymphoma (DLBCL), DLBCL NOS, DLBCL NOS germinal center B cell type (GCB), DLBCL NOS activated B cell type (ABC), primary DLBCL of the central nervous system (CNS), secondary CNS lymphoma (SCNSL), peripheral T cell lymphoma (PCTL), cutaneous T cell lymphoma (CTCL), Sézary syndrome, anaplastic large T-cell lymphoma (ALCL) and anaplastic large T-cell lymphoma, ALK⁺ (ALK⁺ ALCL).

Suitably, said lymphoid neoplasm may be a mature B-cell neoplasm.

Suitably, said mature B-cell neoplasm may be selected from the group consisting of plasma cell myeloma (multiple myeloma), mantle cell lymphoma, Burkitt lymphoma, diffuse large B cell lymphoma (DLBCL), DLBL NOS, DLBCL NOS germinal center B cell type (GCB), DLBCL NOS activated B cell type (ABC), primary DLBCL of the central nervous system (CNS) and secondary CNS lymphoma (SCNSL).

Suitably, said mature B-cell neoplasm may be selected from the group consisting of plasma cell myeloma (multiple myeloma), primary DLBCL of the central nervous system (CNS) and secondary CNS lymphoma (SCNSL).

Suitably, said lymphoid neoplasm may be a neoplasm with primary or secondary central nervous system (CNS) manifestation.

Suitably, said lymphoid neoplasm may be a secondary CNS lymphoma (SCNSL).

Suitably, said lymphoid neoplasm may be a primary DLBCL of the central nervous system (CNS).

Suitably, said lymphoid neoplasm may be a mature T-cell neoplasm.

Suitably, said mature T-cell neoplasm may be selected from the group consisting of peripheral T cell lymphoma (PTCL), PTCL NOS, anaplastic large T-cell lymphoma (ALCL), anaplastic large T-cell lymphoma, ALK⁺ (ALK⁺ ALCL), cutaneous T cell lymphoma (CTCL), Sézary syndrome, and a primary cutaneous CD30+ T cell lymphoproliferative disorder.

Suitably, said mature T-cell neoplasm may be a cutaneous T cell lymphoma (CTCL).

Suitably, said satraplatin may be formulated for oral administration.

Suitably, said satraplatin may be for administration at a dosage schedule of 60-140mg/m² po day 1-5 q 35 days, preferably at a dosage schedule of 80mg/m² po day 1-5 q 35 days.

Alternatively, said satraplatin may be for administration at a dosage schedule of 60-80 mg/m² po day 1-5 q28 days.

Suitably, said treatment may result in killing or inhibition of the growth of tumor cells.

Suitably, said satraplatin may be for administration with one or more additional therapeutic agents.

Suitably, the one or more additional therapeutic agents may be for co-administration.

Suitably, the lymphoid neoplasm may be a C5 type or MCD subtype diffuse large B cell lymphoma (DLBCL).

Suitably, the lymphoid neoplasm may be C5 type or MCD DLBCL with primary or secondary central nervous system (CNS) manifestation.

Suitably, the mammal may be a human having concordant MYD88L265P and CD79B mutations.

Suitably, the human may have additional ETV6, PIM1, GRHPR, TBL1XR1 and PRDM1 mutations.

Further aspects and embodiments of the present invention will be become apparent as this description continues.

### DESCRIPTION OF FIGURES

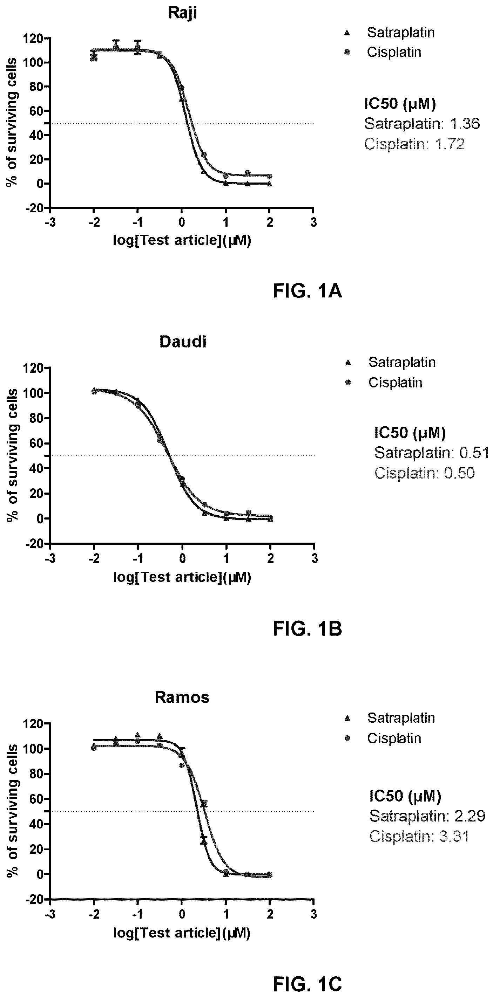

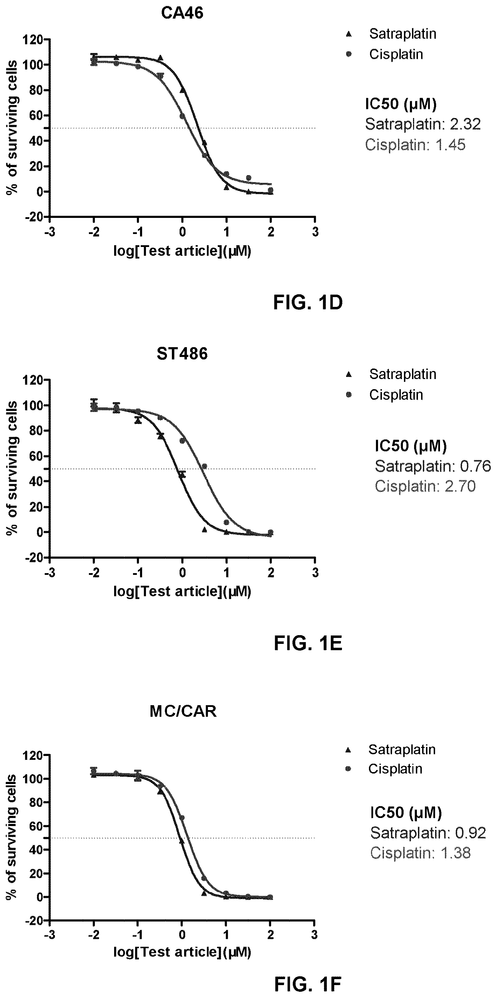

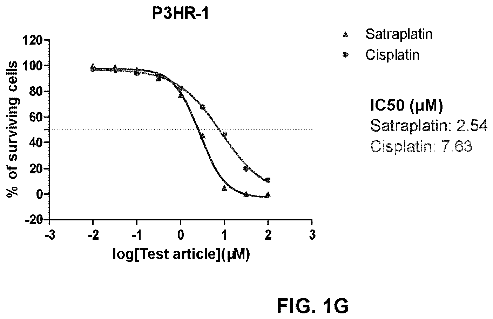

Figures 1A-1G: Effect of satraplatin in comparison to cisplatin on cell viability after a 72h incubation with different concentrations ranging from 10 nM to 100 uM was determined including the IC50 for B-cell lymphoma cell lines, including Burkitt lymphoma cell lines: Raji (FIG. 1A), Daudi (FIG. 1B), Ramos (FIG. 1C), CA46 (FIG. 1D), ST486 (FIG. 1E), MC/CAR (FIG. 1F) and P3HR-1 (FIG. 1G).

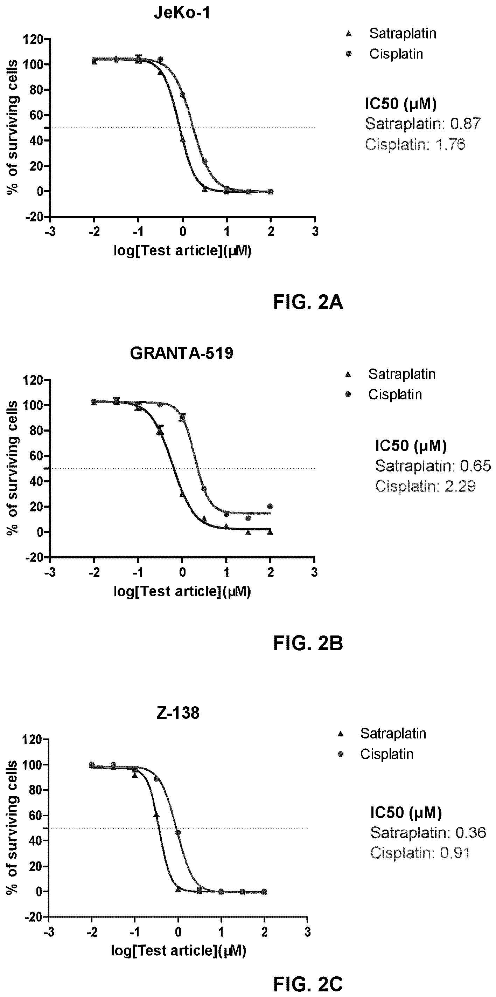

Figures 2A-2C: Effect of satraplatin in comparison to cisplatin on cell viability after a 72h incubation with different concentrations ranging from 10 nM to 100 uM was determined including the IC50 for B-cell lymphoma cell lines, including mantle cell lymphoma cell lines: JeKo-1 (FIG. 2A), GRANTA-519 (FIG. 2B) and Z-138 (FIG. 2C).

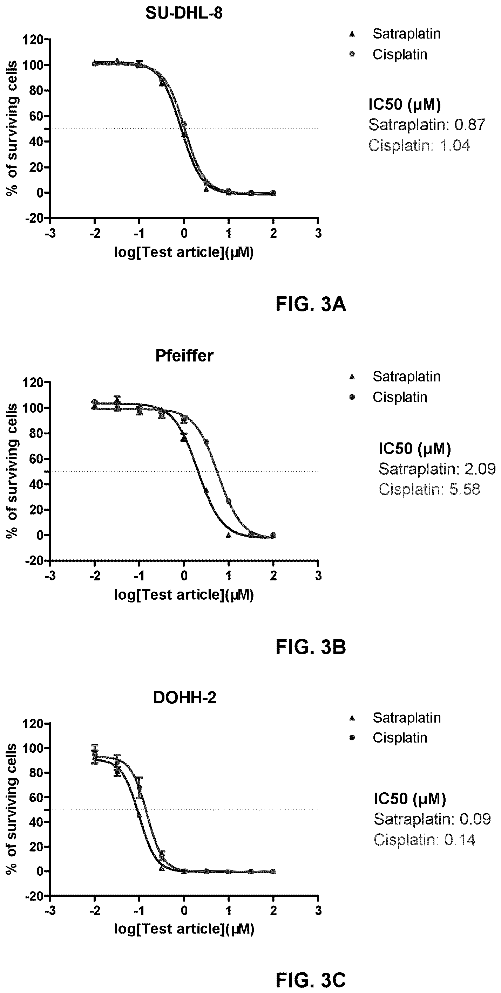

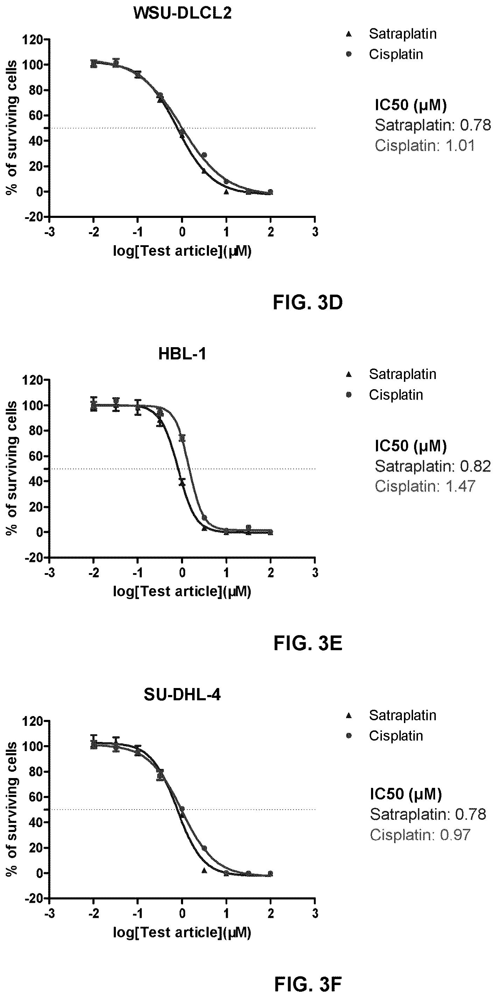

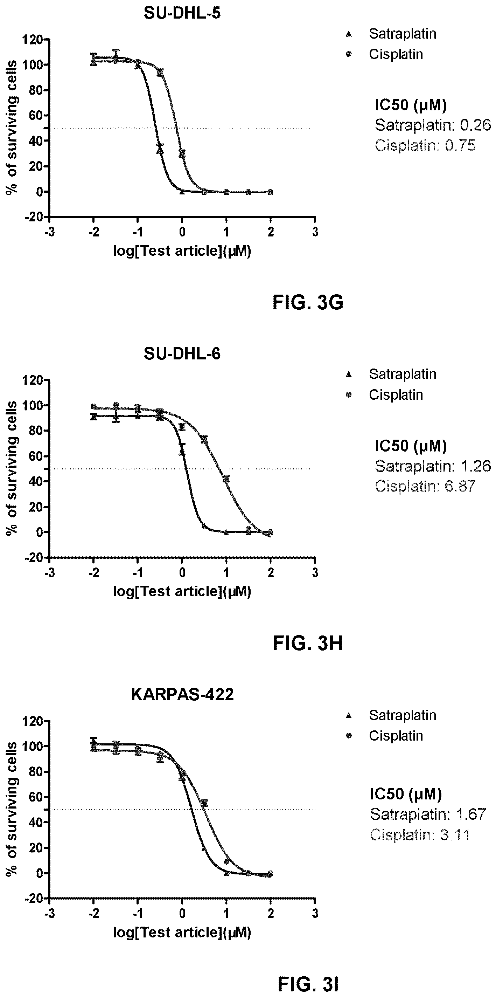

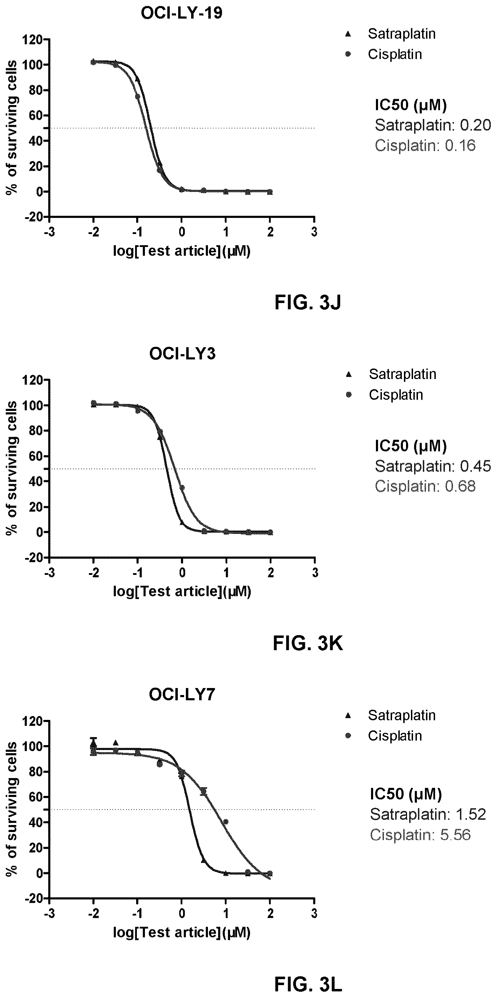

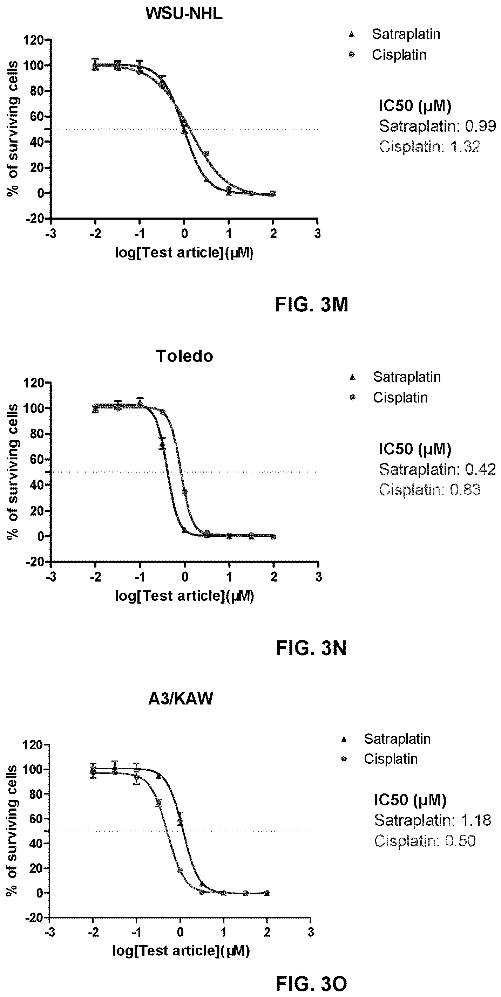

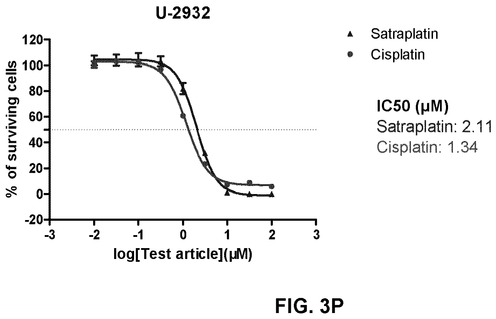

Figures 3A-3P: Effect of satraplatin in comparison to cisplatin on cell viability after a 72h incubation with different concentrations ranging from 10 nM to 100 uM was determined including the IC50 for B-cell lymphoma cell lines, including DLBCL subtype cell lines: SU-DHL-8 (FIG. 3A), Pfeiffer (FIG. 3B), DOHH-2 (FIG. 3C), WSU-DLCL2 (FIG. 3D), HBL-1 (FIG. 3E), SU-DHL-4 (FIG. 3F), SU-DHL-5 (FIG. 3G), SU-DHL-6 (FIG. 3H), KARPAS-422 (FIG. 3I), OCI-LY-19 (FIG. 3J), OCI-LY-3 (FIG. 3K), OCI-LY-7 (FIG. 3L), WSU-NHL (FIG. 3M), Toledo (FIG. 3N), AK/KAW (FIG. 3O) and U-2932 (FIG. 3P).

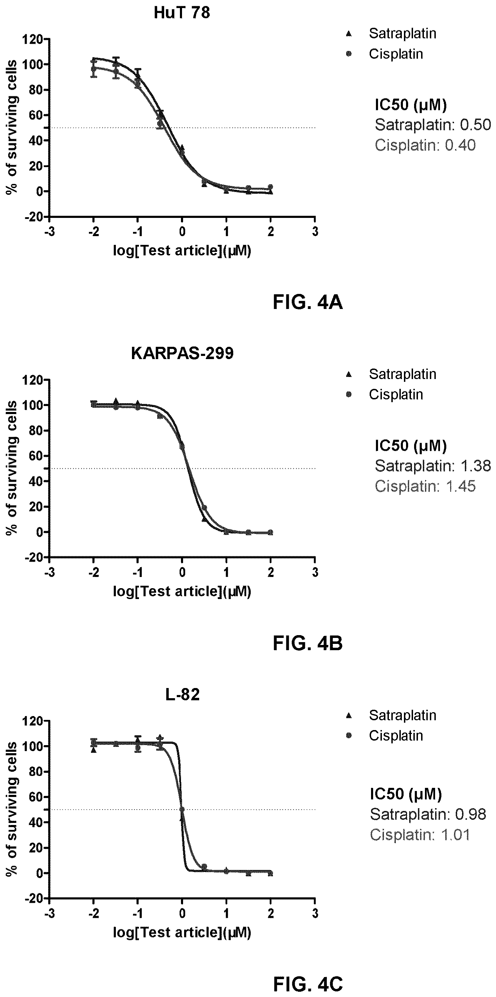

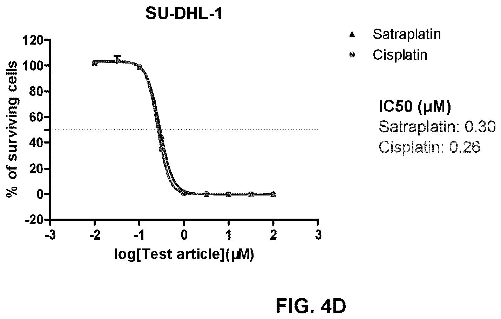

Figures 4A-4D: Effect of satraplatin in comparison to cisplatin on cell viability after a 72h incubation with different concentrations ranging from 10 nM to 100 uM was determined including the IC50 for T-cell lymphoma cell lines: HuT 78 (FIG. 4A), KARPAS-299 (FIG. 4B), L-82 (FIG. 4C) and SU-DHL-1 (FIG. 4D).

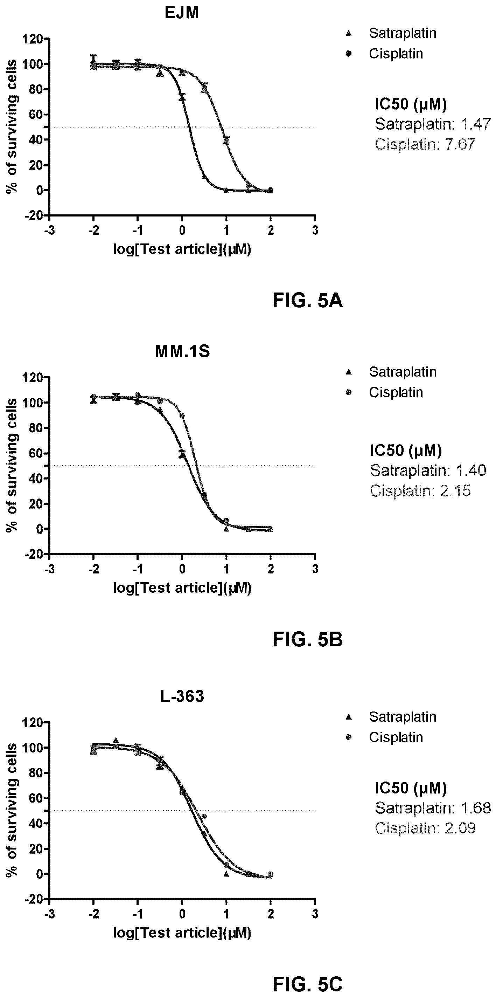

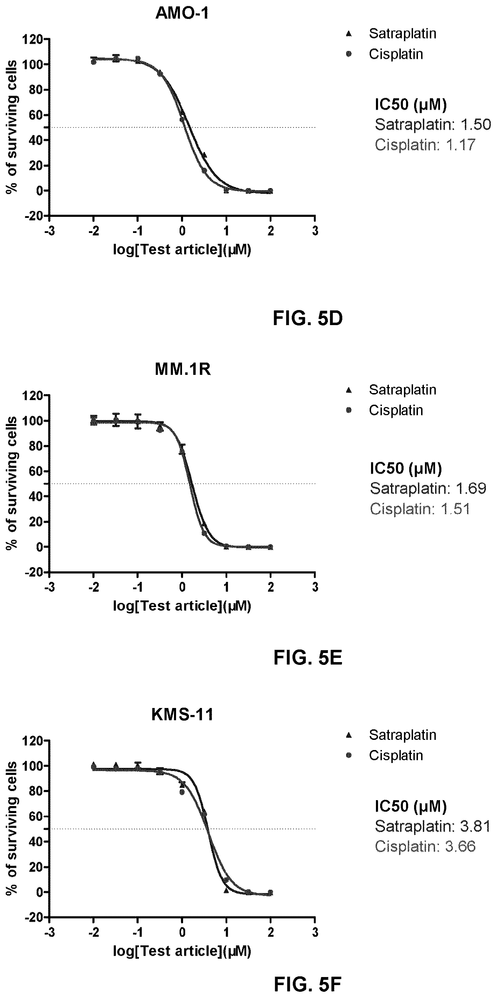

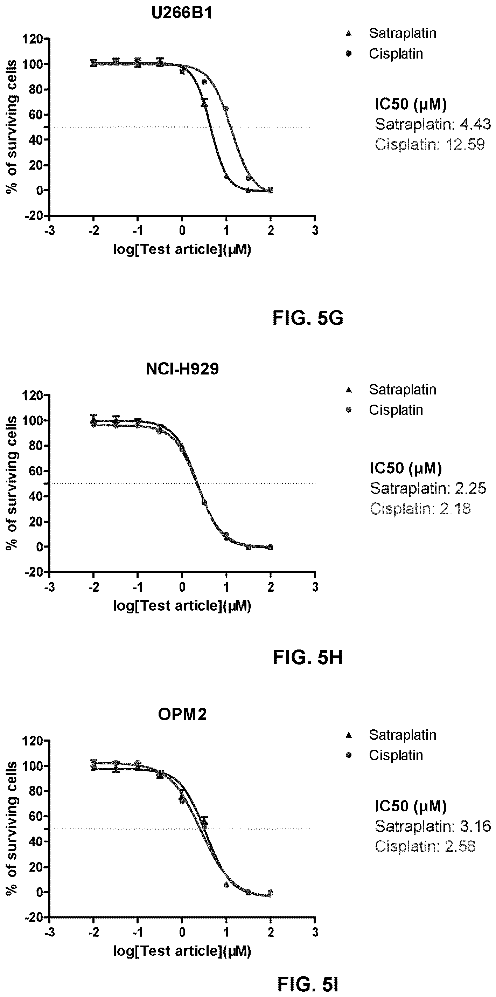

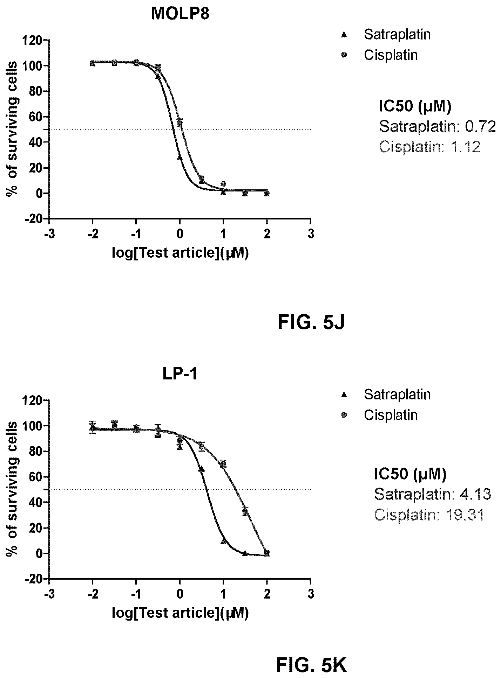

Figures 5A-5K: Effect of satraplatin in comparison to cisplatin on cell viability after a 72h incubation with different concentrations ranging from 10 nM to 100 uM was determined including the IC50 for multiple myeloma cell lines: EJM (FIG. 5A), MM.1S (FIG. 5B), L-363 (FIG. 5C), AMO-1 (FIG. 5D), MM.1R (FIG. 5E), KMS-11 (FIG. 5F), U266B1 (FIG. 5G), NCI-H929 (FIG. 5H), OPM2 (FIG. 5I), MOLP8 (FIG. 5J) and LP-1 (FIG. 5K).
Figure 6: Relative sensitivity of different cell lines to satraplatin.
Figure 7: Genes upregulated in platinum sensitive cell lines (cutoff: FC > 2 & p-value < 0.01)

### DETAILED DESCRIPTION OF THE INVENTION

Unless defined otherwise, all technical and scientific terms used herein have the same meanings as commonly understood by one of ordinary skill in the art to which this invention belongs. The herein described and disclosed embodiments, preferred embodiments and very preferred embodiments should apply to all aspects and other embodiments, preferred embodiments and very preferred embodiments irrespective of whether is specifically again referred to or its repetition is avoided for the sake of conciseness.

The articles "a" and "an", as used herein, refer to one or to more than one (i.e., to at least one) of the grammatical object of the article. The term "or", as used herein, should be understood to mean "and/or", unless the context clearly indicates otherwise. The words "comprise," "comprising," "include," "including," and "includes" when used herein are intended to specify the presence of stated features, integers, components, or steps, but they do not preclude the presence or addition of one or more other features, integers, components, steps, or groups thereof.

The present invention shows that satraplatin is at least equivalent to cisplatin in killing lymphoma and multiple myeloma cell lines *in-vitro.* Moreover, none of the tested cell lines showed a resistance to satraplatin. Furthermore, the present invention shows that satraplatin was effective in killing genetically heterogeneic lymphoma cell lines including those resembling primary or secondary CNS lymphoma. Thus, further considering hereby that satraplatin is orally available, and thus convenient for patients and clinical use, and has furthermore a favourable toxicity profile and is lipophilic with a clinically proven meaningful CNS penetration (Bhargava A and Vaishampayan UN Expert Opin Investig Drugs (2009) 18:1787-1797), the present inventors propose and postulate the use of satraplatin for the treatment of patients with a malignancy of the lymphoid tissue, in particular of human patients with a malignancy of the lymphoid tissue with primary or secondary central nervous system (CNS) manifestation such as, and very preferably, with a primary CNS lymphoma (PCNSL).

Thus, in a first aspect, the present invention provides satraplatin for use in a method of treating a malignancy of the lymphoid tissue in a mammal, preferably a human, and in particular provides satraplatin for the treatment of patients, preferably human patients, with a primary CNS lymphoma (PCNSL).

Satraplatin: Satraplatin (INN), also known as JM-216, or bis (acetato) ammine dichloro (cyclohexylamine) platinum (IV), is a member of a class of platinum (IV) compounds that are absorbed by the oral route. The lipophilic properties of these compounds, and hence their absorption, is believed to be largely determined by the nature of the axial acetate ligands. Unlike the square planar platinum (II) complexes cisplatin and carboplatin, satraplatin is an octahedral platinum (IV) compound. The molecular formula for satraplatin is C₁₀H₂₂N₂Cl₂O₄Pt. Its molecular weight is 500.29 Da. Satraplatin can be synthesized according to the method disclosed in US Patent 5,072,011 and 5,244,919 or by appropriate modification of the method disclosed in US 6,518,428. Its chemical structure is:

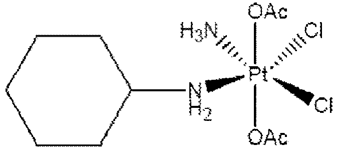

Blood cells mature over several intermediate steps. Stem cells differentiate into cells of either a lymphoid or a myeloid lineage. This results in different clinical presentations, clinical courses, molecular characteristics and different efficacy of drugs.

The term "lymphoid cell" refers to differentiated (or mature) lymphocytes (including T cells and B cells), NK cells and plasma cells. The term "lymphoid tissue" refers to tissue of the lymphatic system (e.g. lymph nodes, spleen, tonsil, or thymus tissue) where the tissue comprises lymphoid cells.

The constant flow of new knowledge led to the 2016 revision of the World Health Organization (WHO) classification of tumours of haematopoietic and lymphoid tissues, in which a distinction was made between haematopoetic neoplasms (such as myeloid neoplasms and acute leukemia) and lymphoid neoplasms (such as lymphomas and multiple myeloma). It defines these neoplasms as separate entities. The respective changes that were made by WHO are published in two different highly cited separate reviews for "lymphoid neoplasms" (Swerdlow SH, Campo E, Pileri SA, et al. The 2016 revision of the World Health Organization classification of lymphoid neoplasms. Blood 2016;127(20):2375-90) and "myeloid neoplasms and acute leukemia" (Arber DA, Orazi A, Hasserjian R, et al. The 2016 revision to the World Health Organization classification of myeloid neoplasms and acute leukemia. teaBlood 2016; 127(20):2391-405).

Malignancy of the lymphoid tissue: The term "malignancy of the lymphoid tissue" refers to a cancer or hyperproliferative disorder generated during hematopoiesis involving cells such as lymphocytes, natural killer cells and plasma cells. The term therefore encompasses abnormal growth of lymphoid cells (i.e. differentiated (or mature) lymphocytes (including T cells and B cells), NK cells and/or plasma cells) or lymphoid tissue (e.g. lymph nodes, spleen, tonsil, or thymus tissue, where the tissue comprises lymphoid cells). Abnormal growth of cells or tissue is also referred to as "neoplasm" herein. A malignancy of lymphoid cells or lymphoid tissue may therefore also be referred to as a "lymphoid neoplasm" herein. The phrases "malignancy of the lymphoid tissue" and "lymphoid neoplasm" are used interchangeably herein, unless the context specifically indicates otherwise.

Lymphoid neoplasms include T cell neoplasms, B cell neoplasms, NK cell neoplasms and plasma cell neoplasms. Lymphoid neoplasms include the mature T cell neoplasms, mature B cell neoplasms and mature NK neoplasms listed below. The lymphoid neoplasms recited herein are well known to a person of skill in the art. They are classified in accordance with the 2016 revision of the World Health Organization (WHO) Classification of lymphoid neoplasms discussed briefly above (Swerdlow SH et al. Blood (2016) 127:2375-2390). The terms "malignancy of the lymphoid tissue" and "lymphoid neoplasm" are used herein in accordance with said WHO classification. As would be clear to a person of skill in the art, the term "lymphoid neoplasm" encompasses lymphoid malignancies such as lymphomas (e.g. T cell lymphoma or B cell lymphoma) and multiple myeloma (plasma cell myeloma, derived from the B cell lineage). It does not encompass myeloid neoplasm nor acute leukemia.

Specific malignancies as well as preferred malignancies in accordance with the present invention are named herein according to the terminology used in said WHO classification. Thus, specific malignancies include the following:
I. Mature B cell neoplasms
   - small lymphocytic lymphoma
   - Monoclonal B cell lymphocytosis
   - Splenic marginal zone lymphoma
   - Lymphoplasmacytic lymphoma
   - Waldenström macroglobulinemia
      ▪ Monoclonal gammopathy of undetermined significance (MGUS), IgM
   - µ heavy chain disease
   - γ heavy chain disease
   - α heavy chain disease
   - Monoclonal gammopathy of undetermined significance (MGUS), IgG/A
   - Plasma cell myeloma (multiple myeloma; the terms are interchangeably used herein)
   - Solitary plasmacytoma of bone
   - Extraosseous plasmacytoma
   - Monoclonal immunoglobulin deposition diseases
   - Extranodal marginal zone lymphoma of mucosa-associated lymphoid tissue (MALT lymphoma)
   - Nodal marginal zone lymphoma
   - Follicular lymphoma
      ▪ *In-situ* follicular neoplasia
      ▪ Duodenal-type follicular lymphoma
   - Pediatric-type follicular lymphoma
   - Primary cutaneous follicle center lymphoma
   - Mantle cell lymphoma
      ▪ In situ mantle cell neoplasia
   - Diffuse large B cell lymphoma (DLBCL), NOS
      ▪ Germinal center B cell type
      ▪ Activated B cell type
   - T cell/histiocyte-rich large B cell lymphoma
   - Primary DLBCL of the central nervous system (CNS)
   - Primary cutaneous DLBCL, leg type
   - EBV+ DLBCL, NOS
   - DLBCL associated with chronic inflammation
   - Lymphomatoid granulomatosis
   - Primary mediastinal (thymic) large B cell lymphoma
   - Intravascular large B cell lymphoma
   - ALK+ large B cell lymphoma
   - Plasmablastic lymphoma
   - Primary effusion lymphoma
   - Burkitt lymphoma
   - High-grade B cell lymphoma
   - High-grade B cell lymphoma, NOS
   - B cell lymphoma, unclassifiable, with features intermediate between DLBCL and classical Hodgkin lymphoma
II. Mature T and NK cell neoplasms
   - Systemic EBV+ T cell lymphoma of childhood
   - Hydroa vacciniforme-like lymphoproliferative disorder
   - Adult T cell lymphoma
   - Extranodal NK/T cell lymphoma, nasal type
   - Enteropathy-associated T cell lymphoma
   - Monomorphic epitheliotropic intestinal T cell lymphoma
   - Hepatosplenic T cell lymphoma
   - Subcutaneous panniculitis-like T cell lymphoma
   - Mycosis fungoides
   - Sézary syndrome
   - Primary cutaneous CD30+ T cell lymphoproliferative disorders
      ▪ Lymphomatoid papulosis
      ▪ Primary cutaneous anaplastic large cell lymphoma
   - Primary cutaneous γδ T cell lymphoma
   - Peripheral T cell lymphoma (PTCL)
      ▪ Peripheral T cell lymphoma (PTCL), NOS
      ▪ Angioimmunoblastic T cell lymphoma
      ▪ Anaplastic large cell lymphoma, ALK⁺ (ALK⁺ ALCL)
      ▪ Anaplastic large cell lymphoma, ALK⁻ (ALK⁻ ALCL)
III. Hodgkin lymphoma
   - Nodular lymphocyte predominant Hodgkin lymphoma
   - Classical Hodgkin lymphoma
      ▪ Nodular sclerosis classical Hodgkin lymphoma
      ▪ Lymphocyte-rich classical Hodgkin lymphoma
      ▪ Mixed cellularity classical Hodgkin lymphoma
      ▪ Lymphocyte-depleted classical Hodgkin lymphoma
IV. Post-transplant lymphoproliferative disorders (PTLD)
   - Plasmacytic hyperplasia PTLD
   - Infectious mononucleosis PTLD
   - Florid follicular hyperplasia PTLD
   - Polymorphic PTLD
   - Monomorphic PTLD (B and T/NK cell types)
   - Classical Hodgkin lymphoma PTLD
V. Histiocytic and dendritic cell neoplasms
   - Histiocytic sarcoma
   - Langerhans cell histiocytosis
   - Langerhans cell sarcoma
   - Indeterminate dendritic cell tumor
   - Interdigitating dendritic cell sarcoma
   - Follicular dendritic cell sarcoma
   - Fibroblastic reticular cell tumor
   - Disseminated juvenile xanthogranuloma
   - Erdheim-Chester disease

Mature B cell neoplasms (including B cell lymphomas, and multiple myeloma, which affects plasma cells that are derived from the B cell lineage) and mature T cell neoplasms (including T cell lymphomas) are particularly relevant to the present invention. The invention is particularly relevant to lymphomas (e.g. T cell and B cell lymphomas). It is also particularly relevant to lymphoid neoplasms with primary or secondary central nervous system (CNS) manifestation such as primary CNS lymphoma (PCNSL).

The term "lymphoma" refers to a lymphoid neoplasm or cancer wherein the affected cells (e.g. T cells, B cells or NK cells) tend to aggregate and form masses, or tumours, in lymphatic tissue. The term "multiple myeloma" refers to a lymphoid neoplasm or cancer wherein the affected cells (plasma cells) tend to aggregate and form masses, or tumours, in bone marrow. The term "leukemia" refers to a haematopoetic neoplasm wherein the affected cells are found circulating in the blood and bone marrow.

A lymphoid neoplasm may also be referred to as a lymphoid cancer herein. The terms "cancer" and "cancerous" refer to or describe the physiological condition in mammals, preferably in humans, that is typically characterized by unregulated increase in cell number (generally referred to herein as cell growth), which can be due to abnormal increase in cell proliferation, abnormal decrease of cell death, or an imbalance of amounts of cell proliferation and cell death.

The term "hyperproliferative" refers to disorders that are associated with some degree of abnormal cell proliferation. In one embodiment, a hyperproliferative disorder is cancer.

CNS lymphoma can either occur as CNS-only manifestation known as primary CNS lymphoma (PCNSL) or as secondary manifestation of a systemic lymphoma that has spread to the central nervous system, known as secondary CNS lymphoma (SCNSL).

Primary CNS lymphoma (PCNSL): The term "primary CNS lymphoma (PCNSL)", as used herein, refers to a malignant lymphoma of the diffuse large B cell (DLBCL) type confined to the CNS. Even though most PCNSLs are diffuse large B-Cell lymphomas (DLBCL; 90%) and share characteristics with systemic DLBCLs, PCNSL is still a unique WHO entity. The terms "primary CNS lymphoma (PCNSL)" and "primary DLBCL of the central nervous system (CNS)" are interchangeably used herein (Swerdlow SH et al. Blood (2016) 127:2375-2390; Deckert M et al Acta Neuropathol (2014) 127:175-188).

Secondary CNS lymphoma (SCNSL): The term "secondary CNS lymphoma (SCNSL) refers to central nervous system spread of a lymphoma that originated outside the CNS (in contrast to primary CNS lymphoma). It can be an indolent or aggressive lymphoma and maybe an isolated recurrence or may be part of a systemic disease at the time of presentation (Tomita N., Kodama F., Kanamori H., Motomura S., Ishigatsubo. Secondary central nervous system lymphoma. Int J Hematol. 2006; 84: 128-135).

The terms "treating" and "treatment" refer to both therapeutic treatment and prophylactic or preventative measures, wherein the object is to prevent or slow down (lessen) an undesired physiological change or disorder, such as the growth, development or spread of cancer. Those in need of treatment include those already with the disorder as well as those prone to have the disorder or those in whom the disorder is to be prevented. For purposes of this invention, beneficial or desired results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment.

The term "therapeutically effective amount" means an amount of satraplatin that (i) treats the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In the case of a malignancy of the present invention, the therapeutically effective amount of satraplatin may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent satraplatin may prevent growth and/or kill existing cancer cells, it may be cytostatic and/or cytotoxic, as measured typically and preferably by the time to disease progression (TTP) and/or determining the response rate (RR).

"Mammal" for purposes of the treatment of, alleviating the symptoms of a malignancy in accordance with the present invention refers to any animal classified as a mammal, including humans, domestic and farm animals, and zoo, sports, or pet animals, such as dogs, cats, cattle, horses, sheep, pigs, goats, rabbits, preferably human, dogs, cats and horses. Further preferably, the mammal is a human. In the first aspect, the present invention provides satraplatin for use in a method of treating a malignancy of the lymphoid tissue in a mammal, preferably a human. The present invention therefore provides satraplatin for use in a method of treating a lymphoid neoplasm in a mammal, preferably a human. Thus, the present invention provides satraplatin for use in a method of treating a lymphoma in a mammal, preferably a human, wherein the method comprising administering a therapeutically effective amount of satraplatin to said mammal, preferably said human. Thus, in a preferred embodiment, said mammal is a human, a dog, a cat or a horse. In a further very preferred embodiment, said mammal is a human.

In a preferred embodiment, said malignancy is a lymphoid neoplasm.

In a preferred embodiment, said malignancy is selected from the group consisting of (i) a mature B-cell neoplasm, (ii) a mature T or NK-cell neoplasm, (iii) a Hodgkin lymphoma, (iv) a post-transplant lymphoproliferative disorder (PTLD) and (v) a histiocytic or dendritic cell neoplasm.

In another preferred embodiment, said malignancy is selected from the group consisting of a mature B-cell neoplasm, a mature T-cell neoplasm and a NK-cell neoplasm.

In another preferred embodiment, said mature B-cell neoplasm, said mature T-cell neoplasm or said NK-cell neoplasm is selected from the group consisting of small lymphocytic lymphoma, monoclonal B cell lymphocytosis, splenic marginal zone lymphoma, lymphoplasmacytic lymphoma, Waldenström macroglobulinemia, monoclonal gammopathy of undetermined significance (MGUS) IgM, µ heavy chain disease, γ heavy chain disease, α heavy chain disease, monoclonal gammopathy of undetermined significance (MGUS) IgG/A, plasma cell myeloma (multiple myeloma), solitary plasmacytoma of bone, extraosseous plasmacytoma, monoclonal immunoglobulin deposition diseases, extranodal marginal zone lymphoma of mucosa-associated lymphoid tissue (MALT lymphoma), nodal marginal zone lymphoma, follicular lymphoma, in situ follicular neoplasia, duodenal-type follicular lymphoma, pediatric-type follicular lymphoma, primary cutaneous follicle center lymphoma, mantle cell lymphoma, *in-situ* mantle cell neoplasia, diffuse large B cell lymphoma (DLBCL) not otherwise specified (NOS), DLBCL NOS germinal center B cell type (GCB), DLBCL NOS activated B cell type (ABC), T cell/histiocyte-rich large B cell lymphoma, primary DLBCL of the central nervous system (CNS), primary cutaneous DLBCL leg type, Epstein-Barr virus (EBV)+DLBCL, secondary CNS lymphoma (SCNSL), DLBCL associated with chronic inflammation, lymphomatoid granulomatosis, primary mediastinal (thymic) large B cell lymphoma, intravascular large B cell lymphoma, ALK+ large B cell lymphoma, plasmablastic lymphoma, primary effusion lymphoma, Burkitt lymphoma, high-grade B cell lymphoma, systemic EBV+ T cell lymphoma of childhood, hydroa vacciniforme-like lymphoproliferative disorder, adult T cell lymphoma, extranodal NK/T cell lymphoma, nasal type, enteropathy-associated T cell lymphoma, monomorphic epitheliotropic intestinal T cell lymphoma, hepatosplenic T cell lymphoma, subcutaneous panniculitis-like T cell lymphoma, mycosis fungoides, Sézary syndrome, primary cutaneous CD30+ T cell lymphoproliferative disorders, lymphomatoid papulosis, primary cutaneous anaplastic large cell lymphoma, cutaneous T cell lymphoma (CTCL), primary cutaneous γδ T cell lymphoma, peripheral T cell lymphoma (PTCL) NOS, angioimmunoblastic T cell lymphoma, anaplastic large cell lymphoma, ALK⁺ (ALK⁺ ALCL), and anaplastic large cell lymphoma, ALK⁻ (ALK⁻ ALCL).

In another preferred embodiment, said lymphoid neoplasm does not include: T cell prolymphocytic leukemia, T cell large granular lymphocytic leukemia, aggressive NK cell leukemia, adult T cell leukemia, chronic lymphoctyic leukemia, B cell prolymphocytic leukemia, or hairy cell leukemia.

In another preferred embodiment, said mature B-cell neoplasm, said mature T-cell neoplasm or said NK-cell neoplasm is selected from the group consisting of plasma cell myeloma (multiple myeloma), follicular lymphoma, in situ follicular neoplasia, duodenal-type follicular lymphoma, mantle cell lymphoma, in situ mantle cell neoplasia, Burkitt lymphoma, diffuse large B cell lymphoma (DLBCL) NOS, DLBCL NOS germinal center B cell type, DLBCL NOS activated B cell type, primary DLBCL of the central nervous system (CNS), secondary CNS lymphoma (SCNSL), peripheral T cell lymphoma (PTCL), NOS, anaplastic large T-cell lymphoma, ALK⁺ (ALK⁺ ALCL) and cutaneous T cell lymphoma (CTCL).

In another preferred embodiment, said mature B-cell neoplasm, said mature T-cell neoplasm or said NK-cell neoplasm is selected from the group consisting of follicular lymphoma, in situ follicular neoplasia, duodenal-type follicular lymphoma, mantle cell lymphoma, in situ mantle cell neoplasia, Burkitt lymphoma, diffuse large B cell lymphoma (DLBCL) NOS, DLBCL NOS germinal center B cell type, DLBCL NOS activated B cell type, primary DLBCL of the central nervous system (CNS), secondary CNS lymphoma (SCNSL), peripheral T cell lymphoma (PTCL), NOS, anaplastic large T-cell lymphoma, ALK⁺ (ALK⁺ ALCL) and cutaneous T cell lymphoma (CTCL).

In another preferred embodiment, said malignancy is a mature B-cell neoplasm or a mature T-cell neoplasm.

In another very preferred embodiment, said mature B-cell neoplasm or said mature T-cell neoplasm is selected from the group consisting of Burkitt lymphoma, plasma cell myeloma (multiple myeloma), mantle cell lymphoma, diffuse large B cell lymphoma (DLBCL) not otherwise specified (NOS), DLBCL NOS germinal center B cell type (GCB), DLBCL NOS activated B cell type (ABC), primary DLBCL of the central nervous system (CNS), secondary CNS lymphoma (SCNSL), peripheral T cell lymphoma (PTCL), PTCL NOS, anaplastic large T-cell lymphoma (ALCL), anaplastic large T-cell lymphoma, ALK⁺ (ALK⁺ ALCL), cutaneous T cell lymphoma (CTCL), Sézary syndrome, and a primary cutaneous CD30+ T cell lymphoproliferative disorder. In one example, the peripheral T cell lymphoma (PTCL) may be PTCL NOS. For example, the PTCL NOS may be anaplastic large T-cell lymphoma (ALCL) or anaplastic large T-cell lymphoma, ALK⁺ (ALK⁺ ALCL). In another example, the cutaneous T cell lymphoma (CTCL) may be Sézary syndrome.

In one example, said mature B-cell neoplasm or said mature T-cell neoplasm is selected from the group consisting of Burkitt lymphoma, mantle cell lymphoma, diffuse large B cell lymphoma (DLBCL) not otherwise specified (NOS), DLBCL NOS germinal center B cell type (GCB), DLBCL NOS activated B cell type (ABC), primary DLBCL of the central nervous system (CNS), secondary CNS lymphoma (SCNSL), peripheral T cell lymphoma (PTCL), PTCL NOS, anaplastic large T-cell lymphoma (ALCL), anaplastic large T-cell lymphoma, ALK⁺ (ALK⁺ ALCL), cutaneous T cell lymphoma (CTCL), Sézary syndrome, and a primary cutaneous CD30+ T cell lymphoproliferative disorder. In one example, the peripheral T cell lymphoma (PTCL) may be PTCL NOS. For example, the PTCL NOS may be anaplastic large T-cell lymphoma (ALCL) or anaplastic large T-cell lymphoma, ALK⁺ (ALK⁺ ALCL). In another example, the cutaneous T cell lymphoma (CTCL) may be Sézary syndrome. In another preferred embodiment, said malignancy is a mature B-cell neoplasm. In one example, the mature B-cell neoplasm is a lymphoma or multiple myeloma. In a specific example, the mature B cell neoplasm is a lymphoma.

In another preferred embodiment, said malignancy is a mature B-cell neoplasm selected from the group consisting of small lymphocytic lymphoma, monoclonal B cell lymphocytosis, splenic marginal zone lymphoma, lymphoplasmacytic lymphoma, Waldenström macroglobulinemia, monoclonal gammopathy of undetermined significance (MGUS) IgM, µ heavy chain disease, γ heavy chain disease, α heavy chain disease, monoclonal gammopathy of undetermined significance (MGUS) IgG/A, plasma cell myeloma (multiple myeloma), solitary plasmacytoma of bone, extraosseous plasmacytoma, monoclonal immunoglobulin deposition diseases, extranodal marginal zone lymphoma of mucosa-associated lymphoid tissue (MALT lymphoma), nodal marginal zone lymphoma, follicular lymphoma, in situ follicular neoplasia, duodenal-type follicular lymphoma, pediatric-type follicular lymphoma, primary cutaneous follicle center lymphoma, mantle cell lymphoma, *in-situ* mantle cell neoplasia, diffuse large B cell lymphoma (DLBCL) NOS, DLBCL NOS germinal center B cell type (GCB), DLBCL NOS activated B cell type (ABC), T cell/histiocyte-rich large B cell lymphoma, primary DLBCL of the central nervous system (CNS), primary cutaneous DLBCL leg type, Epstein-Barr virus (EBV)+DLBCL NOS, secondary CNS lymphoma (SCNSL), DLBCL associated with chronic inflammation, lymphomatoid granulomatosis, primary mediastinal (thymic) large B cell lymphoma, intravascular large B cell lymphoma, ALK+ large B cell lymphoma, plasmablastic lymphoma, primary effusion lymphoma, Burkitt lymphoma, high-grade B cell lymphoma.

In another preferred embodiment, said mature B-cell neoplasm neoplasm does not include: chronic lymphoctyic leukemia, B cell prolymphocytic leukemia, or hairy cell leukemia.
In another very preferred embodiment, said malignancy is a mature B-cell neoplasm selected from the group consisting of plasma cell myeloma (multiple myeloma), follicular lymphoma, in situ follicular neoplasia, duodenal-type follicular lymphoma, mantle cell lymphoma, in situ mantle cell neoplasia, Burkitt lymphoma, diffuse large B cell lymphoma (DLBCL) NOS, DLBCL NOS germinal center B cell type (GCB), DLBCL NOS activated B cell type (ABC), primary DLBCL of the central nervous system (CNS) and secondary CNS lymphoma (SCNSL).

In another very preferred embodiment, said malignancy is a mature B-cell neoplasm selected from the group consisting of follicular lymphoma, in situ follicular neoplasia, duodenal-type follicular lymphoma, mantle cell lymphoma, in situ mantle cell neoplasia, Burkitt lymphoma, diffuse large B cell lymphoma (DLBCL) NOS, DLBCL NOS germinal center B cell type (GCB), DLBCL NOS activated B cell type (ABC), primary DLBCL of the central nervous system (CNS) and secondary CNS lymphoma (SCNSL).

In another very preferred embodiment, said mature B-cell neoplasm is selected from the group consisting of Burkitt lymphoma, plasma cell myeloma (multiple myeloma), mantle cell lymphoma, diffuse large B cell lymphoma (DLBCL) not otherwise specified (NOS), DLBCL NOS germinal center B cell type (GCB), DLBCL NOS activated B cell type (ABC), primary DLBCL of the central nervous system (CNS) and secondary CNS lymphoma (SCNSL).

In another example, said mature B-cell neoplasm is selected from the group consisting of Burkitt lymphoma, mantle cell lymphoma, diffuse large B cell lymphoma (DLBCL) not otherwise specified (NOS), DLBCL NOS germinal center B cell type (GCB), DLBCL NOS activated B cell type (ABC), primary DLBCL of the central nervous system (CNS) and secondary CNS lymphoma (SCNSL).

In a further very preferred embodiment, said mature B cell neoplasm is follicular lymphoma, in situ follicular neoplasia or duodenal-type follicular lymphoma. In a further preferred embodiment, said mature B cell neoplasm is follicular lymphoma, and wherein preferably said follicular lymphoma is in situ follicular neoplasia or duodenal-type follicular lymphoma. In a further very preferred embodiment, said mature B cell neoplasm is mantle cell lymphoma or *in-situ* mantle cell neoplasia. In a further very preferred embodiment, said mature B cell neoplasm is mantle cell lymphoma, wherein said mantle cell lymphoma is in situ mantle cell neoplasia. In a further very preferred embodiment, said mature B cell neoplasm is mantle cell lymphoma. In a further very preferred embodiment, said mature B cell neoplasm is diffuse large B cell lymphoma (DLBCL) NOS, DLBCL NOS germinal center B cell type (GCB) or DLBCL NOS activated B cell type (ABC). In a further very preferred embodiment, said mature B cell neoplasm is diffuse large B cell lymphoma (DLBCL) NOS, wherein said diffuse large B cell lymphoma (DLBCL) NOS is DLBCL NOS germinal center B cell type (GCB) or DLBCL NOS activated B cell type (ABC). In a further very preferred embodiment, said mature B cell neoplasm is diffuse large B cell lymphoma (DLBCL) NOS. In a further very preferred embodiment, said mature B cell neoplasm is diffuse large B cell lymphoma (DLBCL) NOS germinal center B cell type (GBC). In a further very preferred embodiment, said mature B cell neoplasm is diffuse large B cell lymphoma (DLBCL) NOS activated B cell type (ABC). In a further very preferred embodiment, said mature B cell neoplasm is primary DLBCL of the central nervous system (CNS). In a further very preferred embodiment, said mature B cell neoplasm is Burkitt lymphoma.

In a very preferred embodiment, said mature B cell neoplasm is selected from the group consisting of plasma cell myeloma (multiple myeloma), primary DLBCL of the central nervous system (CNS) and secondary CNS lymphoma (SCNSL). In a very preferred embodiment, said mature B cell neoplasm is plasma cell myeloma (multiple myeloma).

In a very preferred embodiment, said mature B cell neoplasm is selected from the group consisting of primary DLBCL of the central nervous system (CNS) and secondary CNS lymphoma (SCNSL).

In a very preferred embodiment, said mature B cell neoplasm is a primary CNS lymphoma (PCNSL) or a secondary CNS lymphoma (SCNSL).

Both, PCNSL and SCNSL, are treated differently than standard DLBCL cases and require high-dose methotrexate (HD-MTX) if possible. However, many CNS lymphoma patients are either ineligible for HD-MTX treatment or will relapse with no standard treatment available at later lines. In particular for these patients, the present invention provides satraplatin as an ideal candidate treatment option and worth to be tested in a prospective clinical trial.

In a further very preferred embodiment, said mature B cell neoplasm is secondary CNS lymphoma (SCNSL). In a further very preferred embodiment, said mature B cell neoplasm is primary CNS lymphoma (PCNSL).

In another preferred embodiment, said malignancy is a lymphoma or a multiple myeloma with primary or secondary CNS manifestation.

In another preferred embodiment, said malignancy is a lymphoma with primary or secondary CNS manifestation.

In another preferred embodiment, said malignancy is a mature T-cell neoplasm or a NK-cell neoplasm. In a preferred embodiment, said mature T-cell neoplasm or said NK-cell neoplasm is selected from the group consisting of systemic EBV+ T cell lymphoma of childhood, hydroa vacciniforme-like lymphoproliferative disorder, adult T cell lymphoma, extranodal NK/T cell lymphoma, nasal type, enteropathy-associated T cell lymphoma, monomorphic epitheliotropic intestinal T cell lymphoma, hepatosplenic T cell lymphoma, subcutaneous panniculitis-like T cell lymphoma, cutaneous T cell lymphoma (CTCL), mycosis fungoides, Sézary syndrome, primary cutaneous CD30+ T cell lymphoproliferative disorders, lymphomatoid papulosis, primary cutaneous anaplastic large cell lymphoma, primary cutaneous γδ T cell lymphoma, peripheral T cell lymphoma (PTCL), PTCL NOS, angioimmunoblastic T cell lymphoma, anaplastic large cell lymphoma (ALCL), anaplastic large cell lymphoma ALK⁺ (ALK⁺ ALCL), and anaplastic large cell lymphoma, ALK⁻(ALK⁻ALCL).
In another preferred embodiment, said mature T-cell neoplasm or a NK-cell neoplasm does not include: T cell prolymphocytic leukemia, T cell large granular lymphocytic leukemia, aggressive NK cell leukemia, or adult T cell leukemia. In a very preferred embodiment, said mature T-cell neoplasma or said NK-cell neoplasm is peripheral T cell lymphoma (PTCL). ).

In another preferred embodiment, said malignancy is a mature T-cell neoplasm.

In another very preferred embodiment, said mature T-cell neoplasm is selected from the group consisting of peripheral T cell lymphoma (PTCL), PTCL NOS, anaplastic large T-cell lymphoma (ALCL), anaplastic large T-cell lymphoma, ALK⁺ (ALK⁺ ALCL), cutaneous T cell lymphoma (CTCL), mycosis fungoides, Sézary syndrome and a primary cutaneous CD30+ T cell lymphoproliferative disorder.

In a preferred embodiment, said mature T-cell neoplasm is a peripheral T cell lymphoma (PTCL), a cutaneous T cell lymphoma (CTCL), mycosis fungoides, Sézary syndrome or a primary cutaneous CD30+ T cell lymphoproliferative disorder.

In another very preferred embodiment, said mature T-cell neoplasm is selected from the group consisting of peripheral T cell lymphoma (PTCL), PTCL NOS, anaplastic large T-cell lymphoma (ALCL), anaplastic large T-cell lymphoma, ALK⁺ (ALK⁺ ALCL), cutaneous T cell lymphoma (CTCL), Sézary syndrome, and primary cutaneous CD30+ T cell lymphoproliferative disorders.

In a very preferred embodiment, said mature T-cell neoplasm is peripheral T cell lymphoma (PTCL) such as PTCL NOS or anaplastic large cell lymphoma (ALCL), anaplastic large cell lymphoma, ALK⁻ (ALK⁻ ALCL) or anaplastic large cell lymphoma ALK⁺ (ALK⁺ ALCL).

In a further very preferred embodiment, said mature T cell neoplasm is peripheral T cell lymphoma (PTCL). In a further very preferred embodiment, said mature T cell neoplasm is peripheral T cell lymphoma (PTCL) NOS. In a further very preferred embodiment, said mature T cell neoplasm is peripheral T cell lymphoma (PTCL) or PTCL NOS. In a further very preferred embodiment, said mature T cell neoplasm is anaplastic large T-cell lymphoma ALCL). In a further very preferred embodiment, said mature T cell neoplasm is anaplastic large T-cell lymphoma, ALK⁺ (ALK⁺ ALCL). In a further very preferred embodiment, said mature T cell neoplasm is anaplastic large T-cell lymphoma. ALK⁻ (ALK⁻ ALCL). In a further very preferred embodiment, said mature T cell neoplasm is cutaneous T cell lymphoma (CTCL), wherein preferably said CTCL is Sézary syndrome or a primary cutaneous CD30+ T cell lymphoproliferative disorder. In a further very preferred embodiment, said mature T cell neoplasm is Sézary syndrome. In a further very preferred embodiment, said mature T cell neoplasm is mycosis fungoides. In a further very preferred embodiment, said mature T cell neoplasm is a primary cutaneous CD30+ T cell lymphoproliferative disorder.

In another very preferred embodiment, said malignancy is a primary cutaneous lymphoma, typically and preferably as defined in the WHO-EORTC classification (Willemze R., et al., Blood 2019; 133(16): 1703-1714), and wherein again further preferably said malignancy is a primary cutaneous T-cell lymphoma (CTCL), typically and preferably as defined in the WHO-EORTC classification (Willemze R., et al., Blood 2019; 133(16): 1703-1714).

In another preferred embodiment, said malignancy is a Hodgkin lymphoma. In a preferred embodiment, said Hodgkin lymphoma is selected from the group consisting of nodular lymphocyte predominant Hodgkin lymphoma, classical Hodgkin lymphoma, nodular sclerosis classical Hodgkin lymphoma, lymphocyte-rich classical Hodgkin lymphoma, mixed cellularity classical Hodgkin lymphoma and lymphocyte-depleted classical Hodgkin lymphoma.

In another preferred embodiment, said malignancy is a post-transplant lymphoproliferative disorder (PTLD). In a preferred embodiment, said post-transplant lymphoproliferative disorder (PTLD) is selected from the group consisting of plasmacytic hyperplasia PTLD, infectious mononucleosis PTLD, florid follicular hyperplasia PTLD, polymorphic PTLD and monomorphic PTLD (B and T/NK cell types).

In another preferred embodiment, said malignancy is a histiocytic or a dendritic cell neoplasm. In a preferred embodiment, said histiocytic and dendritic cell neoplasm is selected from the group consisting of histiocytic sarcoma, Langerhans cell histiocytosis, Langerhans cell sarcoma, indeterminate dendritic cell tumor, interdigitating dendritic cell sarcoma, follicular dendritic cell sarcoma, fibroblastic reticular cell tumor, disseminated juvenile xanthogranuloma and Erdheim-Chester disease.

For use in therapy, satraplatin is typically and preferably suitably formulated in pharmaceutical preparations for oral administration. Thus, in another preferred embodiment, said satraplatin is administered orally.

The skilled person is further aware and knows possible dosages and dosage schedules. It is understood that appropriate doses and dosage schedules depends upon a number of factors known to those or ordinary skill in the art such as a physician. Exemplary doses include milligram or microgram amounts of satraplatin per kilogram of animal, preferably human, subject weight, e. g. about 1 microgram per kilogram to about 500 milligrams per kilogram, about 100 micrograms per kilogram to about 50 milligrams per kilogram, or about milligram per kilogram to about 5 milligrams per kilogram. Thus, further proposed, yet non-limiting dose of satraplatin for administration to an animal, preferably a human, further preferably of a human of 70 kg body weight, may be 0.05 to 2000 mg, preferably 0.1 mg to 1000 mg, of satraplatin per unit dose. The unit dose may be administered, e.g., 1, 2, 3 or more times per day. The unit dose may also be administered 1 to 7 times per week, e.g., with one, two or more administration(s) per day. It will be appreciated that it may be necessary to make routine variations to the dosage depending on the age and weight of the animal or human as well as the severity of the malignancy to be treated.

A person skilled in the art will further appreciate that doses can also be calculated on a body surface basis. A person of 70 kg has an approximate body surface area of 1.8 square meter; doses include milligram or microgram amounts of satraplatin per body surface area of animal or human, e. g. about 50 microgram per square meter to about 15 grams per square meter, about 5 milligrams per square meter to about 1.5 grams per square meter, or about 50 milligram per square meter to about 150 milligrams per square meter. The precise dose will ultimately be at the discretion of the attendant physician. Typical and preferred dosage schedules are 80mg/m² po day 1-5 q 35 days, 100mg/m² po day 1-5 q 35 days, 120mg/m² po day 1-5 q 35 days or 140mg/m² po day 1-5 q 35 days or 120, while lower doses such as 20 mg/m² or 40 mg/m² daily in combination therapies are further typical and preferred. In a further preferred embodiment, said satraplatin is administered at a dosage schedule of 80mg/m² po day 1-5 q 35 days.

In an alternative embodiment, a typical and preferred dosage schedule may be 60-80 mg/m² po day 1-5 q 28 days. For example, the dosage schedule of 60mg/m² po day 1-5 q 28 days, or 70mg/m² po day 1-5 q 28 days, or 80mg/m² po day 1-5 q 28 days may be preferred. Suitable dosage schedules are known in the art, for example see Srivandana Akshintala et al., Phase 1 trial and pharmacokinetic study of the oral platinum analog satraplatin in children and young adults with refractory solid tumors including brain tumors. Pediatr Blood Cancer. 2015 Apr;62(4):603-610.

In another preferred embodiment, said treatment results in killing or inhibition of the growth of tumor cells.

In another preferred embodiment, said use and method further comprises administering one or more additional therapeutic agents to said mammal, preferably to said human. In a further preferred embodiment, said satraplatin and the one or more additional therapeutic agents are co-administered to said mammal, preferably to said human. Preferably, said co-administration of said satraplatin and said one or more additional therapeutic agents are at different schedules such as typically and preferably at different time intervals.

Said additional therapeutic agents are known to the skilled person in the art and include immune checkpoint inhibitors, preferably independently selected from agents that inhibit cytotoxic T-lymphocyte-associated protein 4 (CTLA-4), programmed death 1 (PD-1), programmed death ligand 1 (PD-L1), 4-1BB/4-1BBL, OX40/OX-40L, GITR/GITR-L, T-cell immunoglobulin domain, mucin domain 3 (TIM-3) or lymphocyte activation gene-3 (LAG-3). In a preferred embodiment, said immune checkpoint inhibitor is an antibody. Antibodies of immune checkpoint inhibitors are known and include nivolumab, pembrolizumab, atezolizumab, avelumab and durvalumab.

### EXAMPLES

Cisplatin is a longterm standard chemotherapeutic drug for the treatment of a large variety of malignancies and part of many combined treatment protocols including lymphoma and multiple myeloma. The use of cisplatin in these indications is frequently limited by either pre-existing patient specific morbidities or cisplatin-induced morbidities such as neuro- and nephrotoxicity, let alone that cisplatin does not cross the blood-brain barrier efficiently. Thus, the full therapeutic potential of cisplatin cannot be exploited in many patients.

The inventors have explored satraplatin as an alternative to cisplatin. Satraplatin is an orally available platinum compound with no or not relevant nephro- or neurotoxicity in clinical trials.

The inventors first completed a pathway analysis that confirmed the difference in pathway activation and usage between satraplatin and cisplatin. They performed univariate analysis on gene expression, mutation and copy number levels for 66 cell lines and correlated with this cisplatin and/or satraplatin drug efficacy. They found that expression of genes is significantly correlated with drug response. To identify common and unique molecular features for the two compounds, they overlaid the correlation results on gene expression - cell line sensitivity for satraplatin and cisplatin. By merging the identified differentially expressed (DE) genes of cell line sensitivity for satraplatin and cisplatin, they found 24 genes that are commonly upregulated in sensitive cell lines (Figure 7). However, the majority of genes were differentially expressed with 104 unique genes for satraplatin and 60 unique genes for cisplatin, respectively. These data confirm that these compounds have distinct activity profiles. The complexity of these substances does not allow a prediction of the efficacy of satraplatin in lymphoid neoplasms on the basis of existing data for platinum compounds such as cisplatin. These data clearly reveal that different pathways with differentially expressed genes are involved.

The inventors then analyzed the cell killing potential of satraplatin in comparison to cisplatin on several different lymphoma and multiple myeloma cell lines.

The investigation of tumor cell lines is a useful tool to characterize cancer signaling pathways, drug sensitivity profiles, cell growth, and differentiation dynamics and other important aspects of tumor development and treatment. They provide a continuous and easily available cancer model for many types of oncology research. Cell culture experiments have also paved the way for the development and application of new cancer drugs.

### EXAMPLE 1

### Effect of satraplatin on viability of selected cancer cell lines

The effect of satraplatin in comparison to cisplatin on cell viability of 41 selected cancer cell lines was investigated. Hereto, the 50% inhibitory concentration (IC50) was determined in said cancer cell lines using the CellTiter-Glo^{®} (CTG) luminescent cell viability assay after a 72h incubation with different concentrations of the two drugs ranging from 10 nM to 100 uM.

### Material and Methods

### Cell lines and reagents

The cell lines listed in Table 1 were used. All cell lines were cultured in the media supplemented with 10-15% FBS, at a temperature of 37°C, 5% CO₂ and 95% humidity. Cell viability was measured using the CTG Luminescent Cell Viability Assay by standard procedures. Satraplatin was purchased from MedChemExpress (China) and cisplatin from Qilu Pharm (China), respectively. 1 mM solution of both drugs were established for storage at -20°C as previously described (Wosikowski K et al., Cancer Chemother Pharmacol(2007) 60(4):589-600).

**Table 1. Selected cell lines**

| **No.** | **Cell Line Name** | **Malignancy** | **Reference Control** | **Incubation Time** |
|---|---|---|---|---|
| | | Mature B-cell neoplasms | | |
| 1 | Raji | Burkitt Lymphoma | Cisplatin | 72h |
| 2 | Daudi | Burkitt Lymphoma | Cisplatin | 72h |
| 3 | Ramos | Burkitt Lymphoma | Cisplatin | 72h |
| 4 | CA46 | Burkitt Lymphoma | Cisplatin | 72h |
| 5 | ST486 | Burkitt Lymphoma | Cisplatin | 72h |
| 6 | M/CAR | Burkitt Lymphoma | Cisplatin | 72h |
| 7 | P3HR-1 | Burkitt Lymphoma | Cisplatin | 72h |
| 8 | JeKo-1 | Mantle Cell Lymphoma | Cisplatin | 72h |
| 9 | GRANTA-519 | Mantle Cell Lymphoma | Cisplatin | 72h |
| 10 | Z-138 | Mantle Cell Lymphoma | Cisplatin | 72h |
| 11 | SU-DHL-8 | Diffuse large B cell lymphoma (DLBCL) NOS, germinal center B cell type (GCB) | Cisplatin | 72h |
| 12 | Pfeiffer | Diffuse large B cell lymphoma (DLBCL) NOS, germinal center B cell type (GCB) | Cisplatin | 72h |
| 13 | DOHH-2 | Diffuse large B cell lymphoma (DLBCL) NOS, germinal center B cell type (GCB) | Cisplatin | 72h |
| 14 | WSU-DLCL2 | Diffuse large B cell lymphoma (DLBCL) NOS, germinal center B cell type (GCB) | Cisplatin | 72h |
| 15 | HBL-1 | Diffuse large B cell lymphoma (DLBCL) NOS, activated B cell type (ABC) | Cisplatin | 72h |
| 16 | SU-DHL-4 | Diffuse large B cell lymphoma (DLBCL) NOS, germinal center B cell type (GCB) | Cisplatin | 72h |
| 17 | SU-DHL-5 | Diffuse large B cell lymphoma (DLBCL) NOS, germinal center B cell type (GCB) | Cisplatin | 72h |
| 18 | SU-DHL-6 | Diffuse large B cell lymphoma (DLBCL) NOS, germinal center B cell type (GCB) | Cisplatin | 72h |
| 19 | KARPAS-422 | Diffuse large B cell lymphoma (DLBCL) NOS, germinal center B cell type (GCB) | Cisplatin | 72h |
| 20 | OCI-LY-19 | Diffuse large B cell lymphoma (DLBCL) NOS, germinal center B cell type (GCB) | Cisplatin | 72h |
| 21 | OCI-LY-3 | Diffuse large B cell lymphoma (DLBCL) NOS, activated B cell type (ABC) resembling PCNSL by mutational profile | Cisplatin | 72h |
| 22 | OCI-LY-7 | Diffuse large B cell lymphoma (DLBCL) NOS, germinal center B cell type (GCB) | Cisplatin | 72h |
| 23 | WSU-NHL | Diffuse large B cell lymphoma (DLBCL) NOS, germinal center B cell type (GCB) | Cisplatin | 72h |
| 24 | Toledo | Diffuse large B cell lymphoma (DLBCL) NOS, germinal center B cell type (GCB) | Cisplatin | 72h |
| 25 | A3/KAW | Diffuse large B cell lymphoma (DLBCL) NOS | Cisplatin | 72h |
| 26 | U-2932 | Diffuse large B cell lymphoma (DLBCL) NOS, activated B cell type (ABC) | Cisplatin | 72h |
| 27 | EJM | Multiple Myeloma | Cisplatin | 72h |
| 28 | MM.1S | Multiple Myeloma | Cisplatin | 72h |
| 29 | L-363 | Multiple Myeloma | Cisplatin | 72h |
| 30 | AMO-1 | Multiple Myeloma | Cisplatin | 72h |
| 31 | MM.1R | Multiple Myeloma | Cisplatin | 72h |
| 32 | KMS-11 | Multiple Myeloma | Cisplatin | 72h |
| 33 | U266B1 | Multiple Myeloma | Cisplatin | 72h |
| 34 | NCI-H929 | Multiple Myeloma | Cisplatin | 72h |
| 35 | OPM2 | Multiple Myeloma | Cisplatin | 72h |
| 36 | MOLP8 | Multiple Myeloma | Cisplatin | 72h |
| 37 | LP-1 | Multiple Myeloma | Cisplatin | 72h |

| | | Mature T-cell neoplasms | | |
|---|---|---|---|---|
| 38 | HuT 78 | Cutaneous T-cell lymphoma (CTCL), Sézary lymphoma | Cisplatin | 72h |
| 39 | KARPAS-299 | Peripheral T cell lymphoma (PTCL), anaplastic large cell lymphoma, ALK+ (ALK⁺ ALCL) | Cisplatin | 72h |
| 40 | L-82 | Peripheral T cell lymphoma (PTCL), anaplastic large cell lymphoma (ALCL) | Cisplatin | 72h |
| 41 | SU-DHL-1 | Peripheral T cell lymphoma (PTCL), anaplastic large cell lymphoma , ALK+ (ALK⁺ ALCL) | Cisplatin | 72h |

### Determination of the half maximal inhibition concentration IC50

Cells were harvested during the logarithmic growth period and count cell number using Count-star. Cell concentrations were adjusted to 4.44× 10⁴ cells/mL with the respective culture medium. Then, 90 µL cell suspensions were added in triplicate to two 96-well plates (plates A and B) with the final cell density of 4× 10³ cells/well. After 24 hours, 10× solutions of cisplatin and satraplatin starting with 100 µM in media with 3.16-fold serial dilutions to achieve 9 dose levels were established on a separate plate. Then, 10 µL (10×) drug solution of either satraplatin or cisplatin were transferred in each well (triplicate for each drug concentration) of the plate B. The test plate B was incubated for 72 h in the humidified incubator at 37°C with 5% CO₂, and then measured by means of CTG assay. For that purpose, plates were equilibrated at RT for approximately 30 min. Then, 50 µL CTG reagent was added to each well. The content was mixed for 5 min on an orbital shaker to induce cell lysis and the plate incubated at RT for 20 min to stabilize luminescent signal. Luminescence was recorded using EnVision Multi Label Reader.

### Data Analysis

The data are displayed graphically using GraphPad Prism 5.0. In order to calculate absolute IC50 (EC50), a dose-response curve was fitted using a nonlinear regression model with a sigmoidal dose response. The formula for calculating surviving rate is: The surviving rate (%) = (LumTest article-LumMedium control)/(LumNon-treated-LumMedium control)× 100%. The absolute IC50 (EC50) was calculated according to the dose-response curve generated by GraphPad Prism 5.0. The standard deviation was calculated for each dose level and each cell line and is indicated by the bars.

### Results

A plurality of different human lymphoma and myeloma cell lines were used to provide evidence for the efficacy of satraplatin and were tested for their sensitivity to be killed by satraplatin, with cisplatin as reference, as described. Killing of lymphoma cell lines was very effective (Figures 1-6) with IC 50 values at low micromolar concentrations or even lower.

The data presented herein shows that satraplatin kills lymphoma cancer cells in a dose dependent manner similar or superior to cisplatin.

For example, the data shows that satraplatin was at least equivalent to cisplatin at inducing cell death of cell lines representative of B-cell lymphoma, particularly Burkitt lymphoma (FIG. 1A to FIG. 1G), mantle cell lymphoma (FIG. 2A to FIG. 2C), and DLBCL (FIG. 3A to FIG. 3P).

Satraplatin was also at least equivalent to cisplatin in inducing cell death of cell lines representative of T-cell lymphoma, particularly anaplastic large-cell lymphoma, ALK-positive (ALK⁺ ALCL) (FIG. 4A to FIG. 4D)

Finally, satraplatin was also shown to be at least equivalent to cisplatin in inducing cell death of cell lines representative of multiple myeloma (FIG. 5A to FIG. 5L).

All of the cell lines described herein are sensitive to satraplatin. However, as shown in Figure 6, the level of sensitivity varied per cell line. As shown therein, certain cell lines were particularly sensitive to satraplatin treatment. These cell lines are representative of mature B cell neoplasms (e.g. DLBCL (DOHH2, OCI Ly19, SUDHL5, OCI Ly3, Toledo cell lines), mantle cell lymphoma (Z138 cell line), Burkitt lymphoma (Daudi cell line)) and mature T cell neoplasms (e.g. ALK+ ALCL (SUDHL1 cell line), CTCL (HuT78 cell line)). Satraplatin may therefore be particularly useful when treating DLBCL, mantle cell lymphoma, Burkitt lymphoma, CTCL and ALK+ ALCL.

A number of the cell lines used herein are representative of a wider group of malignancies. By way of example only, the HUT 78 cell line was derived from a patient suffering from the most aggressive form of cutaneous T-cell lymphoma (Sézary-Syndrome). Surprisingly, Satraplatin showed one of the best efficacies measured in the experiments described herein in this cell line (IC 50 0.3µM). Exceptional activity in other aggressive T-cell lines that occur in cutaneous variants was also observed (e.g. PCALCL - primary cutaneous anaplastic large cell lymphoma). SU-DHL1 with an IC 0.3 µM is a good example (anaplastic T-cell lymphoma). The data generated for HUT 78 is therefore representative of the efficacy that is expected when satraplatin is used across the entire spectrum of cutaneous T-cell lymphomas.

The *in vitro* data presented herein is predictive of the efficacy of satraplatin treatment *in vivo.* Satraplatin has previously been extensively tested in different cell lines of solid tumors (Wosikowski K, Lamphere L, Unteregger G, et al. Preclinical antitumor activity of the oral platinum analog satraplatin. Cancer Chemother Pharmacol 2007;60(4):589-600). As an example, experiments in nude mice bearing human prostate tumor xenocrafts (Sova P, Mistr A, Kroutil A, Zak F, Pouckova P, Zadinova M. Comparative anti-tumor efficacy of two orally administered platinum(IV) drugs in nude mice bearing human tumor xenografts. Anticancer Drugs 2006;17(2):201-6) confirmed the efficacy of the compound very well in *in-vivo* models. On the basis of these experiments, clinical development in prostate carcinoma was advanced. Subsequently, clinical development of the drug was initiated in metastatic prostate cancer. In the SPARC trial, 25% of patients receiving satraplatin showed a relevant PSA-response (decline of more than 50%) (Sternberg CN, Petrylak DP, Sartor O, et al. Multinational, double-blind, phase III study of prednisone and either satraplatin or placebo in patients with castrate-refractory prostate cancer progressing after prior chemotherapy: The SPARC trial. J Clin Oncol 2009;27(32):5431-8). This confirmed the presumed efficacy, which had already become apparent by the previously performed *in-vitro* experiments. For satraplatin, *in-vitro* experiments therefore provide good evidence of efficacy in humans. *In-vitro* efficacy can therefore be used as a predictive marker for anticipated *in-vivo* efficacy.

### EXAMPLE 2

### Mutational pattern of cell line OCI Ly-3

As described before, DLBCL cell lines were very sensitive to both, cisplatin and satraplatin, at lC50 values at low micromolar or even sub-molar doses. Moreover, satraplatin was not only significantly superior in killing DLBCL cell lines such as OCI-LY7 cells when compared to cisplatin, but in addition and importantly, satraplatin killed very efficiently the cell line OCI-LY3 (FIG. 3K).

The efficacy on OCI LY-3 cell lines is in particular relevant. The OCI Ly-3 cell line has been sequenced twice from different laboratories with an estimated 2-year culturing duration difference between samples. Despite this, the same mutations were found and no differential mutations were detected. OCI Ly-3 showed the highest amount of consistency in the comparative analysis. Mutations found were MYD88, PIM1, CD79B, CARD11 and PRDM1 (Table 2). These mutations resemble the genetic landscape of the Cluster 5 DLBCL lymphoma indicative for PCSNL as recently described by Chapuy et al. (Chapuy B et al. Nature Medicine, (2018) 24:679-690). Chapuy et al. described the genetic features and mutational signatures of newly characterized DLBCL subsets and clusters (C1-C5). Particularly relevant is the mentioned C5 signature that includes concordant MYD88L265P/CD79B mutations, and additional mutations of ETV6, PIM1, GRHPR, TBL1XR1 and PRDM1. These mutations are typical for primary CNS lymphoma and they found that eight of nine PCNSL patients were in this C5 cluster (Fisher's exact test, P<0.001). These data laid the basis for the acceptance of said C5 genetic signature resembling PCNSL as further confirmed by profiling 48 subjects with PCNSL from 2013-2018 at the MD Anderson Cancer Center, Houston, Texas (Ou, A et al. Neuro-Oncology Advances (2020) 2:1-8).

Therefore, and based on the mutational pattern, OCI Ly-3 is the most accepted PCNSL-like human DLBCL cell line, and, as indicated, highly suitable to predict and compare the efficacy of an investigational compound for the treatment of PCNSL. As shown in FIG. 3K, OCI Ly-3 was very effectively killed by satraplatin.

**Table 2: Mutational pattern of OCI Ly-3.**

| **Mutation** | **C5 Signature*** | **OCI LY-3 Cell Line** | **PCNSL (profiling of 48 patients)** |
|---|---|---|---|
| MYD88 | + | + | 81% |
| PIM1 | + | + | 55% |
| CD79B | + | + | 55 % |
| CARD 11 | - | + | 10% |
| ETV6 | + | 9 | 10 % |
| PRDM1 | + | + | 5% |

| | | | |
|---|---|---|---|
| * Chapuy B et al. Nature Medicine (2018) 24:679-690. ** Ou, A et al. Neuro-Oncology Advances (2020) 2:1-8. | | | |

In view of the OCI Ly-3 data presented herein, satraplatin is particularly useful in the treatment of a C5 type or MCD subtype diffuse large B cell lymphoma (DLBCL). In this context, a C5 type DLBCL is as defined in Chapuy B et al. Nature Medicine, (2018) 24:679-690. Similarly, a MCD subtype DLBCL is as defined in Wright et al., Cancer cell, (2020) 37, 551-568. The clinical presentations of C5 type and MCD subtype DLBCL are well characterized in the field. A person of skill in the art can therefore readily identify such subjects for treatment with satraplatin.

MCD DLBCL tumors are known to spread secondarily to extranodal sites in 30% of cases, and 46% of these occurred at sites that can give rise to primary extranodal lymphomas-the central nervous system (CNS), vitreo-retina, testis, and breast-whereas other DLBCL subtypes spread to these sites significantly less often. This category of DLBCL is represented by the cell lines OCI-Ly-3 and HBL-1 (Wright et al, 2020). In view of the data presented herein, it is therefore clear that satraplatin is particularly useful in the treatment DLBCL with primary extranodal lymphoma presentation, for example with lymphoma of the central nervous system (CNS), vitreo-retina, testis, and/or breast. Of particular interest is the use of satraplatin in the treatment of C5 type or MCD subtype DLBCL with primary or secondary central nervous system (CNS) manifestation.

As is known by a person of skill in the art, C5 and MCD DLBCL are primarily defined by MYD88L265P and CD79B mutations. In view of the data presented herein, satraplatin is therefore particularly useful in the treatment of lymphoid neoplasms (e.g. C5 or MCD DLBCL) in humans having concordant MYD88L265P and CD79B mutations. Such mutations and means for detecting such mutations are well known in the art. A person of skill in the art can therefore readily identify such subjects for treatment with satraplatin.

In the context of the C5 signature, concordant MYD88L265P/CD79B mutations, and additional mutations of ETV6, PIM1, GRHPR, TBL1XR1 and PRDM1 have been observed. In view of the data presented herein, satraplatin is therefore particularly useful in the treatment of C5 DLBCL in humans having concordant MYD88L265P and CD79B mutations, optionally with additional ETV6, PIM1, GRHPR, TBL1XR1 and PRDM1 mutations. Such mutations and means for detecting such mutations are well known in the art. A person of skill in the art can therefore readily identify such subjects for treatment with satraplatin.

The invention further includes the subject matter of the claims of WO2022008149 from which this application is derived, the content of which is reproduced below as numbered paragraphs (paras).
1. Satraplatin for use in treating a lymphoid neoplasm in a mammal.
2. Satraplatin for use according to para 1, wherein said mammal is a human.
3. Satraplatin for use according to para 1 or 2, wherein said lymphoid neoplasm is a mature B-cell neoplasm or a mature T-cell neoplasm.
4. Satraplatin for use according to para 3, wherein said mature B-cell neoplasm or said mature T-cell neoplasm is selected from the group consisting of: plasma cell myeloma (multiple myeloma), mantle cell lymphoma, Burkitt lymphoma, diffuse large B cell lymphoma (DLBCL), DLBCL NOS, DLBCL NOS germinal center B cell type (GCB), DLBCL NOS activated B cell type (ABC), primary DLBCL of the central nervous system (CNS), secondary CNS lymphoma (SCNSL), peripheral T cell lymphoma (PCTL), cutaneous T cell lymphoma (CTCL), Sézary syndrome, anaplastic large T-cell lymphoma (ALCL) and anaplastic large T-cell lymphoma, ALK⁺ (ALK⁺ ALCL).
5. Satraplatin for use according to any one of the paras 1 to 4, wherein said lymphoid neoplasm is a mature B-cell neoplasm.
6. Satraplatin for use according to para 5, wherein said mature B-cell neoplasm is selected from the group consisting of plasma cell myeloma (multiple myeloma), mantle cell lymphoma, Burkitt lymphoma, diffuse large B cell lymphoma (DLBCL), DLBL NOS, DLBCL NOS germinal center B cell type (GCB), DLBCL NOS activated B cell type (ABC), primary DLBCL of the central nervous system (CNS) and secondary CNS lymphoma (SCNSL).
7. Satraplatin for use according to para 5, wherein said mature B-cell neoplasm is selected from the group consisting of plasma cell myeloma (multiple myeloma), primary DLBCL of the central nervous system (CNS) and secondary CNS lymphoma (SCNSL).
8. Satraplatin for use according to any of the paras 1 to 7, wherein said lymphoid neoplasm is a neoplasm with primary or secondary central nervous system (CNS) manifestation.
9. Satraplatin for use according to any of the paras 1 to 8, wherein said lymphoid neoplasm is a secondary CNS lymphoma (SCNSL).
10. Satraplatin for use according to any of the paras 1 to 8, wherein said lymphoid neoplasm is a primary DLBCL of the central nervous system (CNS).
11. Satraplatin for use according to any one of the paras 1 to 4, wherein said lymphoid neoplasm is a mature T-cell neoplasm.
12. Satraplatin for use according to para 11, wherein said mature T-cell neoplasm is selected from the group consisting of peripheral T cell lymphoma (PTCL), PTCL NOS, anaplastic large T-cell lymphoma (ALCL), anaplastic large T-cell lymphoma, ALK⁺ (ALK⁺ ALCL), cutaneous T cell lymphoma (CTCL), Sézary syndrome, and a primary cutaneous CD30+ T cell lymphoproliferative disorder.
13. Satraplatin for use according to para 11, wherein said mature T-cell neoplasm is a cutaneous T cell lymphoma (CTCL).
14. Satraplatin for use according to any one the paras 1 to 13, wherein said satraplatin is formulated for oral administration.
15. Satraplatin for use according to para 14, wherein said satraplatin is for administration at a dosage schedule of;
   a) 60-140mg/m² po day 1-5 q 35 days, preferably at a dosage schedule of 80mg/m² po day 1-5 q 35 days; or
   b) 60-80 mg/m² po day 1-5 q 28 days.
16. Satraplatin for use according to any one of the paras 1 to 15, wherein said treatment results in killing or inhibition of the growth of tumor cells.
17. Satraplatin for use according to any one of paras 1 to 16, wherein said satraplatin is for administration with one or more additional therapeutic agents.
18. Satraplatin for use according to para 17, wherein said satraplatin and the one or more additional therapeutic agents are for co-administration.
19. Satraplatin for use according to any preceding para , wherein the lymphoid neoplasm is a C5 type or MCD subtype diffuse large B cell lymphoma (DLBCL).
20. Satraplatin for use according to para 19, wherein the lymphoid neoplasm is DLBCL with primary or secondary central nervous system (CNS) manifestation.
21. Satraplatin for use according to para 19 or 20, wherein the mammal is a human having concordant MYD88L265P and CD79B mutations.
22. Satraplatin for use according to para 21, wherein the human has additional ETV6, PIM1, GRHPR, TBL1XR1 and PRDM1 mutations.

## Claims

1. Satraplatin for use in treating a lymphoid neoplasm in a mammal, wherein said lymphoid neoplasm is a mature B-cell neoplasm or a mature T-cell neoplasm, wherein said mature B-cell neoplasm or said mature T-cell neoplasm is selected from the group consisting of: mantle cell lymphoma, Burkitt lymphoma, diffuse large B cell lymphoma (DLBCL), secondary CNS lymphoma (SCNSL), primary CNS lymphoma (PCNSL), cutaneous T cell lymphoma (CTCL), and a lymphoma with primary or secondary central nervous system (CNS) manifestation.

2. Satraplatin for use according to claim 1, wherein said mature B-cell neoplasm is selected from the group consisting of: DLBCL NOS, DLBCL NOS germinal center B cell type (GCB), DLBCL NOS activated B cell type (ABC), and primary DLBCL of the central nervous system (CNS).

3. Satraplatin for use according to claim 1, wherein said mature B-cell neoplasm is a primary CNS lymphoma (PCNSL).

4. Satraplatin for use according to any of the preceding claims, wherein said lymphoid neoplasm is a lymphoma with primary or secondary central nervous system (CNS) manifestation.

5. Satraplatin for use according to any of the preceding claims, wherein said lymphoid neoplasm is primary CNS lymphoma (PCNSL).

6. Satraplatin for use according to any of the claims 1 or 2, or claim 4, wherein said lymphoid neoplasm is a secondary CNS lymphoma (SCNSL).

7. Satraplatin for use according to any of the claims 1 to 5, wherein said lymphoid neoplasm is a primary DLBCL of the central nervous system (CNS).

8. Satraplatin for use according to claim 1, wherein said mature T-cell neoplasm is selected from the group consisting of: Sézary syndrome, and a primary cutaneous CD30+ T cell lymphoproliferative disorder.

9. Satraplatin for use according to claim 1, wherein said mature T-cell neoplasm is a cutaneous T cell lymphoma (CTCL).

10. Satraplatin for use according to any one the claims 1 to 9, wherein said satraplatin is formulated for oral administration.

11. Satraplatin for use according to claim 10, wherein said satraplatin is for administration at a dosage schedule of;
a) 60-140mg/m² po day 1-5 q 35 days; or
b) 60-80 mg/m² po day 1-5 q 28 days.

12. Satraplatin for use according to claim 11, wherein said satraplatin is for administration at a dosage schedule of 80mg/m² po day 1-5 q 35 days.

13. Satraplatin for use according to any preceding claim, wherein said treatment results in killing or inhibition of the growth of tumor cells.

14. Satraplatin for use according to any preceding claim, wherein said treatment attenuates, ameliorates, or eliminates one or more symptoms of the disease, condition, or disorder.

15. Satraplatin for use according to any preceding claim, wherein said treatment reduces the number of cancer cells; reduces the tumor size; inhibits cancer cell infiltration into peripheral organs; inhibits tumor metastasis; inhibits tumor growth; and/or relieves one or more of the symptoms associated with the cancer.

16. Satraplatin for use according to any preceding claim, wherein said treatment results in killing or inhibition of the growth of cancer cells.

17. Satraplatin for use according to any one of claims 1 to 16, wherein said satraplatin is for administration with one or more additional therapeutic agents.

18. Satraplatin for use according to claim 17, wherein said satraplatin and the one or more additional therapeutic agents are for co-administration.

19. Satraplatin for use according to any preceding claim, wherein said mammal is a human.

20. Satraplatin for use according to any of claims 1-7 or claims 10-19, wherein the lymphoid neoplasm is a C5 type or MCD subtype diffuse large B cell lymphoma (DLBCL).

21. Satraplatin for use according to claim 20, wherein the lymphoid neoplasm is DLBCL with primary or secondary central nervous system (CNS) manifestation.

22. Satraplatin for use according to claim 20 or 21, wherein the mammal is a human having concordant MYD88L265P and CD79B mutations.

23. Satraplatin for use according to claim 22, wherein the human has additional ETV6, PIM1, GRHPR, TBL1XR1 and PRDM1 mutations.
